Europäisches Patentamt

European Patent Office

Office européen des brevets

(19)

(11) Numéro de publication: **0 180 500**
**A1**

(12) **DEMANDE DE BREVET EUROPEEN**


(21) Numéro de dépôt: **85401941.1**

(22) Date de dépôt: **04.10.85**

(51) Int. Cl.⁴: **C 07 D 491/04**, C 07 D 495/04, A 61 K 31/40, A 61 K 31/41 // (C07D491/04, 307:00, 209:00), (C07D495/04, 333:00, 209:00)

(30) Priorité: **12.10.84 FR 8415658**

(43) Date de publication de la demande: **07.05.86 Bulletin 86/19**

(84) Etats contractants désignés: **AT BE CH DE FR GB IT LI LU NL SE**



(71) Demandeur: **LIPHA, LYONNAISE INDUSTRIELLE PHARMACEUTIQUE, 34, rue Saint Romain Boîte Postale 8481, F-69359 Lyon Cedex 08 (FR)**

(72) Inventeur: **Festal, Didier, Pins 5 Charrière Blanche, F-69130 Ecully (FR)**
Inventeur: **Descours, Denis, 59, avenue Galline, F-69100 Villeurbanne (FR)**
Inventeur: **Depin, Jean-Claude, 113, Cours Gambetta, F-69003 Lyon (FR)**
Inventeur: **Quentin, Yvette, 32, cours d'Herbouville, F-69004 Lyon (FR)**

(74) Mandataire: **Bouton Neuvy, Liliane et al, L'Air liquide, Société Anonyme pour L'Etude et L'Exploitation des Procédés Georges Claude 75, Quai d'Orsay, F-75321 Paris Cedex 07 (FR)**


(54) **Dérivés de thiéno et furo - [2,3-c]pyrroles, procédés de préparation et médicaments les contenant.**


(57) Thiéno et furo-[2,3-c]-pyrroles représentés par la formule:

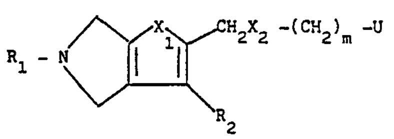

dans laquelle $X_1$ et $X_2$ désignent un atome d'oxygène ou de soufre; $R_1$ désigne un radical alkyle linéaire ou ramifié contenant de 1 à 8 atomes de carbone, un radical alcynyle, un radical alkoxyalkyle contenant de 3 à 8 atomes de carbone ou un reste aromatique; $R_2$ désigne un atome d'hydrogène ou un radical alkyle linéaire ou ramifié; m = 2 ou 3, quand m = 2, $X_1$ et $X_2$ désignent un atome de soufre, U représente un reste diamino-3,4-cyclobuténdione-1,2 ou aminosulfonylformamidine; U peut aussi représenter les structures azotées

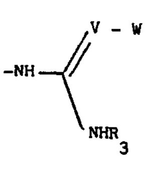 ; 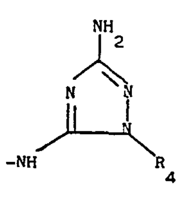 ; 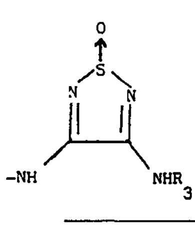

dans lesquelles V désigne un atome d'azote ou un fragment CH; W représente $NO_2$ ou CN; $R_3$ désigne un atome d'hydrogène, un radical alkyle linéaire ou ramifié de 1 à 8 atomes de carbone, un radical alcynyle; $R_4$ désigne un groupement alkyle contenant de 1 à 3 atomes de carbone.

Médicament à action antagoniste des récepteurs $H_2$ de l'histamine et antiulcéreuse contenant comme principe actif un thiéno ou furo-[2,3-c]-pyrrole.


EP 0 180 500 A1

DESCRIPTION

La présente invention concerne des dérivés du dihydro-5, 6-4H-thiéno [2,3-c] pyrrole et du dihydro-5,6-4H-furo [2,3-c] pyrrole, les procédés de préparation de ces composés, et les médicaments les contenant.

L'histamine présente dans de nombreux tissus animaux, est impliquée dans plusieurs mécanismes physiologiques, par exemple la sécrétion acide de l'estomac. L'histamine agit en se fixant sur des récepteurs tissulaires qui ont été subdivisés en récepteurs de type H1, bloqués par les médicaments anti histaminiques classiques telle la Prométhazine et en récepteurs de type H2 insensibles à ce type de médicaments et responsables notamment de la sécrétion acide de l'estomac.

Divers composés ont été proposés comme antagonistes des récepteurs H2 à l'histamine et par là de l'acidité gastrique et présentés comme principes actifs de compositions médicamenteuses utiles dans le traitement des ulcères gastriques et autres affections provoquées ou exacerbées par l'acidité gastrique et notamment la Cimétidine et la Ranitidine.

De multiples revues et monographies ont déjà été consacrées à la classe de composés de ce type qui continue de faire l'objet de nombreux travaux visant à trouver de nouveaux composés plus actifs et mieux adaptés au traitement de la maladie ulcéreuse.

Chimiquement, la comparaison des composés décrits, montre qu'ils sont généralement constitués d'un cycle aromatique benzénique ou hétérocyclique, relié par l'intermédiaire d'une chaîne aliphatique comportant le plus souvent un hétéroatome, principalement le soufre ou l'oxygène, à un reste choisi parmi les groupements azotés de la chimie organique.

Un nombre relativement faible de composés bicycliques condensés possédant des propriétés anti ulcéreuses par le biais d'une action antogoniste des récepteurs H2 à l'histamine semblent avoir fait l'objet d'études.

Par le brevet U.S. n° 4.238.487 on connait des dérivés du benzofuranne et par le brevet belge 779.775 des imidazopyridines.

La demande de brevet international WO 84/00544 décrit des composés doués de propriétés cytoprotectrices et antihistaminiques $H_2$ définis de façon très large comme pouvant dériver d'un système bicyclique constitué d'un noyau benzénique ou hétérocyclique à 5 ou 6 chaînons accolé à un cycle azoté aromatique ou polyhydrogéné, incluant ainsi des dérivés de furo- et thiénopyridines et d'isoquinoléines.

Les schémas réactionnels et procédés de synthèse présentés dans la partie descriptive ne concernent que des dérivés d'isoquinoléine et de furo- ou thiéno pyridines, et les seuls composés décrits sont des dérivés d'isoquinoléine.

Aucun des schémas réactionnels proposés, ni aucun des procédés de synthèse ne permet de préparer ou ne concerne des systèmes thiéno et furo (2,3-c) pyrroles.

Le brevet français 2.384.765 a pour objet des dérivés aminosubstitués du furanne et le brevet français 2.391.209 des aminoalkylthiophènes.

Les composés décrits dans ces deux brevets ont une structure monocyclique dialkylamino alkyl-furanne ou thiophène.

Ces composés utiles comme médicaments ont un mécanisme d'action classique du type de la "Cimétidine".

En outre, le brevet britannique 2.098.988 intitulé : "1,2-diamino-cyclobuten-3,4-dione derivatives" a pour objet notamment des composés dont la structure associe un cycle thiophénique ou furannique avec une terminaison aminocyclobutènedione.

Ces composés dont le mécanisme d'action antihistaminique est comparable à celui de la "cimétidine", manifeste une activité tout au plus équivalente.

Conformément à la présente invention, on vient de découvrir de façon fortuite que certains dérivés des systèmes bicycliques fusionnés dihydro-5,6-4H-thiéno [2,3-c] pyrrole et dihydro-5,6-4H-furo [2,3-c] pyrrole représentes respectivement par les formules Ia et Ib ci-dessous,

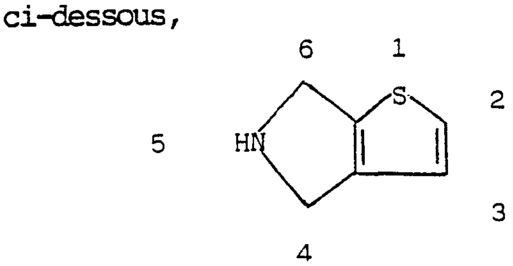

(I a)

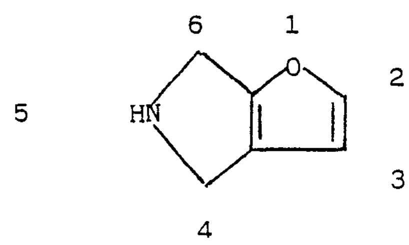

(I b)

possèdent des propriétés antisécrétoires et antiulcéreuses particulièrement intenses et qu'à cet égard ils se sont révélés largement supérieurs aux composés couramment utilisés en thérapeutique comme la Cimétidine ou la Ranitidine ou encore aux dérivés de tétrahydroisoquinoléine décrits comme produits préférés dans la demande de brevet international WO 84/00544 cité ci-dessus, et auxquels ils ont été comparés en raison de la parenté structurale qu'on peut estimer exister entre ces divers composés. De plus, on a trouvé que l'action antagoniste des récepteurs $H_2$ à l'histamine exercée par ces composés procède par un mécanisme original pour cette activité, de type faiblement réversible leur conférant ainsi des propriétés antisécrétoires remarquablement durables.

Les composés de l'invention répondent à la formule,

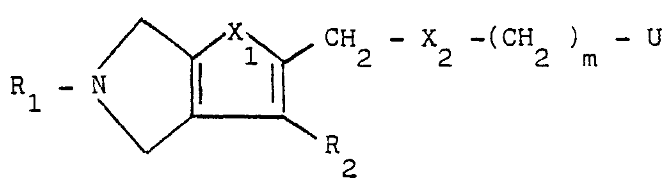

(II)

dans laquelle, $X_1$ et $X_2$ désignent selon le cas un atome d'oxygène ou de soufre ;

$R_1$ désigne un radical alkyle linéaire ou ramifié contenant 1 à 8 atomes de carbone, un radical alcynyle de préférence propargyle, un radical alkoxyalkyle contenant 3 à 8 atomes de carbone ou un reste aromatique tel que phényle ou phénylalkyle de préférence benzyle ; $R_2$ peut désigner un atome d'hydrogène ou un radical alkyle linéaire ou ramifié, de préférence un radical méthyle auquel cas $X_1$ désigne obligatoirement un atome de soufre ; m étant égal à 2, U peut représenter un reste aminosulfonylformamidine ou un reste diamino-3,4 cyclobutène dione-1,2, auxquels cas $X_1$ et $X_2$ désignent tous deux un atome de soufre ; avec m = 2, U peut aussi représenter une structure azotée répondant à la formule générale III ou encore un reste hétérocyclique aminotriazole de formule générale IV ; U peut aussi représenter un reste aminothiadiazole de formule V, avec la condition que lorsque $X_1$ désigne un atome d'oxygène, m soit égal à 2.

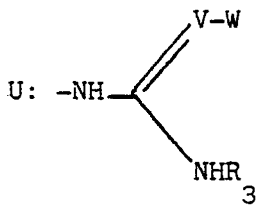
(III)

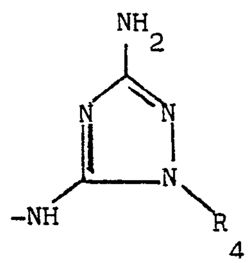
(IV)

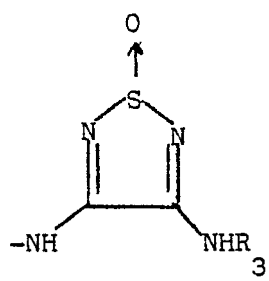
(V)

Dans la formule, (III), V peut représenter un atome d'azote ou un fragment CH, W un groupement de préférence insaturé tel que cyano ou nitro et $R_3$ un atome d'hydrogène, un groupement alkyle linéaire ou ramifié contenant 1 à 8 atomes de carbone, un radical alcynyle de préférence propargyle ; dans la formule (IV), $R_4$ peut désigner un groupement alkyle contenant de 1 à 3 atomes de carbone, mais de préférence un groupe méthyle ; dans la formule (V), $R_3$ possède les significations précédemment définies.

Il convient de noter que dans les formules III à V ci-dessus, bien que les doubles liaisons aient été placées dans des positions particulières, différentes autres formes tautomères sont possibles et que l'invention a également pour objet ces formes tautomères tant pour ce qui

est des composés de l'invention que pour ce qui est des nouveaux intermédiaires de synthèse.

Comme composés spécifiques de l'invention on peut citer :

- N-Cyano N' -[ [ (éthyl-5 dihydro-5,6-4H-thiéno [ 2,3-c ] pyrrolyl-2) méthylthio ] -2 éthyl ] N" -méthyl guanidine
- N- [ [ (Ethyl-5 dihydro-5,6-4H-thiéno [ 2,3-c ] pyrrolyl-2 ) méthylthio ]-2 éthyl ] méthylthio-1 nitro-2 éthénamine
- N- [ [ (Ethyl-5 dihydro-5,6-4H-thiéno [ 2,3-c ] pyrrolyl-2) méthylthio ]-2 éthyl ] N' -méthyl nitro-2 éthène diamine-1,1
- N-Cyano N' - [ [ (éthyl-5 dihydro-5,6-4H-thiéno [ 2,3-c ] pyrrolyl-2) méthylthio ]-2 éthyl ] N"-isopropyl guanidine
- N-Cyano N' - [ [ (dihydro-5,6 méthyl-5-4H-thiéno [ 2,3-c ] pyrrolyl-2) méthylthio ]-2 éthyl ] N" -méthyl guanidine
- N-Cyano N' - [ [ (dihydro-5,6 isopropyl-5-4H-thiéno- [ 2,3-c ] pyrrolyl-2) méthylthio ]-2 éthyl ] N"-méthyl guanidine
- N- [ [(Benzyl-5 dihydro-5,6-4H-thiéno- [ 2,3-c ] pyrrolyl-2)méthylthio] -2 éthyl ] N' -cyano N"-méthyl guanidine
- [ (Ethyl-5 dihydro-5,6-4H-thiéno [ 2,3-c ] pyrrolyl-2) méthylthio ] -3 N-sulfamoyl propanamidine
- N-Cyano N' -éthyl N" - [[ (éthyl-5 dihydro-5,6-4H-thiéno [ 2,3-c ] pyrrolyl-2) méthylthio ] -2 éthyl ] guanidine
- N-[ [ (Ethyl-5 dihydro-5,6-4H-thiéno [ 2,3-c ] pyrrolyl-2) méthylthio ] -2 éthyl ] méthyl-1-1H-triazole-1,2,4 diamine-3,5
- N-Cyano N' - [ [ (dihydro-5,6 phényl-5-4H-thiéno [ 2,3-c ] pyrrolyl-2) méthylthio ] -2 ethyl ] N" -méthyl guanidine
- N- [ [ (Ethyl-5 dihydro-5,6-4H-thiéno [ 2,3-c ] pyrrolyl-2) méthylthio] -2 éthyl ] oxyde-1 thiadiazole [ 1,2,5 ] diamine-3,4
- N- [ [ (Dihydro-5,6 méthyl-5-4H-thiéno [ 2,3-c ] pyrrolyl-2) méthylthio ] -2 éthyl ] N' -méthyl nitro-2 éthène diamine-1,1
- N- [ [ (Ethyl-5 dihydro-5,6-4H-furo [ 2,3-c ] pyrrolyl-2) méthylthio ] -2 éthyl ] N' -méthyl nitro-2 éthène diamine-1,1
- N-Cyano N' - [ [ (éthyl-5 dihydro-5,6-4H-furo [ 2,3-c ] pyrrolyl-2) méthylthio ] -2 éthyl ] N" -méthyl guanidine
- N- [ [ (Ethyl-5 dihydro-5,6-4H-furo [ 2,3-c ] pyrrolyl-2) méthylthio ] -2 éthyl ] méthylthio-1 nitro-2 éthènamine
- N-Cyano N' - [ [ (éthyl-5 dihydro-5,6-4H-thiéno [ 2,3-c ] pyrrolyl -2) méthylthio ] -2 éthyl ] N" propargyl guanidine

- N- [ [ (Dihydro-5,6 n. pentyl-5-4H-thiéno [ 2,3-c ] pyrrolyl-2) méthylthio ]-2 éthyl ] N' -méthyl nitro-2 éthène diamine-1,1
- N- [ [ (Ethyl-5 dihydro-5,6 méthyl-3-4H-thiéno [ 2,3-c ] pyrrolyl -2) méthylthio ] -2 éthyl ] N' -méthyl nitro-2 éthène diamine-1,1
- N- [ [ (Ethyl-5 dihydro-5,6-4H-thiéno [ 2,3-c ] pyrrolyl-2) méthylthio ] -2 éthyl ] N' -méthyl oxyde-1 thiadiazole [ 1,2,5 ] diamine-3,4
- N- [ [(Ethyl-5 dihydro-5,6-4H-thiéno [ 2,3-c ] pyrrolyl-2) méthylthio] -2 éthyl ] N' -propargyl oxyde-1 thiadiazole [ 1,2,5 ] diamine-3,4
- N- [ [ (Dihydro-5,6 n. propyl-5-4H-thiéno [ 2,3-c ] pyrrolyl-2) méthylthio ] -2 éthyl ] oxyde-1 thiadiazole [ 1,2,5 ] diamine-3,4
- N- [ [ (Ethyl-5 dihydro-5,6 méthyl-3-4H-thiéno [ 2,3-c ] pyrrolyl -2) méthylthio ] -2 éthyl ] méthylthio-1 nitro-2 éthènamine
- N- [ [ (Dihydro-5,6 n. pentyl-5-4H-thiéno [ 2,3-c ] pyrrolyl-2) méthylthio ] -2 éthyl ] méthylthio-1 nitro-2 éthènamine
- N-Cyano N' - [ [ (Dihydro-5,6 phényl-5-4H-thiéno [ 2,3-c ] pyrrolyl-2) méthylthio ] -2 éthyl ] S-méthyl isothiourée
- N-Cyano N' - [ [ (éthyl-5 dihydro-5,6-4H-thiéno [ 2,3-c ] pyrrolyl -2) méthylthio ] -2 éthyl ] S-méthyl isothiourée
- N-[ [ (éthyl-5 dihydro-5,6-4H-thiéno [ 2,3-c ] pyrrolyl-2) méthoxy ] -2 éthyl ] oxyde-1 thiadiazole [ 1,2,5 ] diamine-3,4
- N- [ [ (n. Butyl-5 dihydro-5,6-4H-thiéno [ 2,3-c ] pyrrolyl-2) méthylthio ] -2 éthyl ] oxyde-1 thiadiazole [ 1,2,5 ] diamine-3,4
- N- [ [ Isobutyl-5 dihydro-5,6-4H-thiéno [ 2,3-c ] pyrrolyl-2) méthylthio ] -2 éthyl ] oxyde-1 thiadiazole [ 1,2,5 ] diamine-3,4
- N- [ [ (n. Hexyl-5 dihydro-5,6-4H-thiéno [ 2,3-c ] pyrrolyl -2) méthylthio ] -2 éthyl ] oxyde-1 thiadiazole [ 1,2,5 ] diamine-3,4
- N- [ [ (Dihydro-5,6 n.pentyl-5-4H-thiéno [ 2,3-c ] pyrrolyl-2) méthylthio ] -2 éthyl ] oxyde-1 thiadiazole [ 1,2,5 ] diamine-3,4
- N- [ [ (Ethyl-5 dihydro-5,6-4H-thiéno [ 2,3-c ] pyrrolyl -2) méthylthio ] -3 propyl ] oxyde-1 thiadiazole [ 1,2,5 ] diamine-3,4
- N- [ [ [ Dihydro-5,6 (méthoxy-2 éthyl)-5-4H-thiéno [ 2,3-c ] pyrrolyl-2) méthylthio ] -2 éthyl ] oxyde-1 thiadiazole [ 1,2,5 ] diamine-3,4
- Amino-3 [ [ (dihydro-5,6 n.propyl-5-4H-thiéno [ 2,3-c ] pyrrolyl-2) méthylthio ] -2 éthylamino ] -4 cyclobutène dione-1,2
- N- [ [ (Dihydro-5,6 n.propyl-5-4H-furo [ 2,3-c ] pyrrolyl -2) méthylthio ] -2 éthyl ] oxyde-1 thiadiazole [ 1,2,5 ] diamine-3,4

Les composés de l'invention forment facilement des sels d'addition avec un acide minéral ou organique ; comme sels d'acides minéraux acceptables en thérapeutique on peut citer par exemple les chlorhydrates, bromhydrates, sulfates, phosphates, sulfonates, de même des exemples de sels organiques particulièrement utilisés sont les acétates, tartrates, citrates, camphosulfonates, maléates, fumarates, méthanesulfonates ; tous ces sels d'addition rentrent également dans le cadre de l'invention.

En raison de leur capacité à inhiber la sécrétion d'acide gastrique dans l'organisme, les composés sont doués d'activité anti-ulcéreuse ; cette action particulièrement intense et durable rend extrêmement intéressante leur utilisation dans le traitement des ulcères peptiques et autres manifestations pathologiques similaires. Il convient de noter que la durée et l'intensité des actions antisécrétoires et antiulcéreuses des composés de l'invention de formule I sont fonction de la nature des hétéro-atome $X_1$ et $X_2$ mais également pour une grande part de la nature du substituant $R_1$ et qu'à cet égard les composés de la formule I tout particulièrement préférés sont ceux pour lesquels X1 et X2 désignent un atome de soufre et $R_1$ représente un groupement tel que défini précédemment et possédant un fragment aliphatique lié à l'atome d'azote du cycle pyrrole d'au moins deux atomes de carbone.

Une méthode couramment utilisée pour l'étude des antisécrétoires gastriques est constituée par les rats au pylore lié selon H. Shay et Coll., Gastroenterology, 1945, 5 43 : Des rats femelles d'environ 200 g, à jeun de 72 heures, ont le pylore ligaturé sous légère anesthésie à l'éther. Quatre heures plus tard, les animaux sont sacrifiés et le contenu des estomacs prélevé pour mesure de l'acidité. Les composés sont injectés intrapéritonéalement, juste après la ligature du pylore. La dose abaissant l'acidité gastrique de 50 % (DE 50) est calculée pour chaque composé, par rapport à un lot non traité. Chaque composé est testé à 3 doses géométriquement espacées. Chaque lot comprend 10 rats.

Le blocage des récepteurs histaminiques $H_2$ impliqués dans la sécrétion acide de l'estomac a été étudié in vitro sur l'oreillette isolée de cobaye et in vivo par la méthode de l'hyperacidité gastrique à l'histamine chez le rat anesthésié.

Oreillette isolée de cobaye : Des cobayes albinos pesant entre 300 et 400 g sont assommés et saignés. Après ouverture du thorax, l'oreillette droite est rapidement dégagée et placée dans une cuve à organe isolé contenant 100 ml de Krebs (composition en g/1 : NaCl 6,9 - KCl 0,35 - $CaCl_2$ 0,28 - Mg $SO_4$ 0,14 - $NaHCO_3$ 2,09 - $KH_2$ $PO_4$ 0,16, glucose 2) maintenu à 32°C et fortement oxygéné (95 % $O_2$, 5 % $CO_2$).

L'oreillette est reliée à une jauge de contrainte isométrique, sous une tension de 1 g. Les battements spontanés sont enregistrés sur un enregistreur galvanométrique.

Après une période de stabilisation de 1 heure, des doses cumulées d'histamine sont ajoutées au milieu de survie, toutes les 5 minutes, ce qui occasionne une accélération des battements par stimulation des récepteurs de l'histamine qui, dans les tissus cardiaques sont de nature $H_2$. L'histamine est ajoutée jusqu'à ce que le rythme de l'oreillette n'augmente plus (effet maximum). On peut alors tracer la courbe représentant la variation du rythme en fonction de la concentration d'histamine dans le bain. Après rinçages et repos de 30 minutes, la même expérimentation est reprise 30 minutes après avoir ajouté un antagoniste $H_2$ dans le bain (composé de l'invention ou produit de référence).

Chaque composé est ainsi testé à 3 concentrations géométriquement croissantes qui provoquent, en cas de blocage des récepteurs $H_2$, un déplacement vers la droite des courbes concentration d'histamine/effet chronotrope. Pour chaque composé actif, nous déterminons un $pA_2$, c'est à dire le cologarithme de la concentration du composé de l'essai pour laquelle il faut doubler la concentration d'histamine afin de retrouver un effet chronotrope donné. Le calcul est effectué par la méthode de O. Arunlakshana et H.O. Shild, Br. J. Pharmac., 1959, <u>14</u>, 48.
Un minimum de 3 oreillettes est utilisé pour chaque composé essayé.

Cette préparation permet aussi de se rendre compte de la nature, réversible ou non, de l'inhibition. Dans le premier cas, il sera toujours possible de déplacer l'antagoniste des récepteurs en pratiquant des rinçages répétés ou en utilisant une concentration plus forte d'histamine, ce qui se traduira par des courbes concentration d'histamine/effet chronotrope, parallèles mais superposables.

Par contre, dans le deuxième cas (inhibition difficilement réversible), l'antagonisme $H_2$ ne peut être complètement levé, ni par

rinçages ni par des concentrations élevées d'histamine, ce qui se traduit par des courbes dose/effet non superposables (l'effet chronotrope maximum est de plus en plus faible quand la concentration de l'antagoniste $H_2$ croit).

Hyperacidité gastrique à l'histamine chez le rat anesthésié : (Black et Coll., Nature, 1972, 236, 385) : Des rats anesthésiés à l'éthyluréthane reçoivent une perfusion continue d'histamine (25 µg/kg/minute) dans une veine jugulaire. L'estomac est continuellement perfusé par du sérum physiologique à 37°C, sous un débit constant de 3 ml/minute.

Une canule pylorique collecte le perfusat dont l'acidité est mesurée de façon continue. Chaque composé à examiner est administré par la voie intraveineuse (une seule dose par rat). L'inhibition de l'acidité est mesurée au délai correspondant au maximum d'action (délai variable d'un composé à un autre). Un minimum de 3 rats est utilisé pour chaque dose essayée. On détermine, pour chaque composé actif de l'invention, la dose diminuant de 50 % (DE 50), l'hyperacidité induite par l'histamine. On mesure en outre, pour les composés les plus actifs, la durée d'action résultant de l'injection intraveineuse d'une dose unique correspondant à la DE 50 arrondie.

Les composés de l'invention sont en général très peu toxiques. Par exemple, le composé de l'exemple 22 n'exerce aucun effet toxique quand on l'administre en I.V. à des souris ou a des rats, à une dose représentant 100 fois sa DE 50 dans le test de l'hyperacidité gastrique à l'histamine chez le rat anesthésié.

Les tableaux 1 à 4 suivants donnent pour les méthodes décrites ci-dessus les activités des composés de l'invention ainsi que des produits de référence. Il convient de préciser que le composé noté A dans les tableaux suivants, désigne la N-méthyl N' - [(méthyl-2 tétrahydro-1,2,3,4 isoquinoléine-5 oxy)-3 propyl] nitro-2 éthène diamine-1,1 décrite et revendiquée comme composé préféré dans la demande de brevet international WO 84/00544.

Tableau 1

| Rats au pylore lié selon Shay | | | |
|---|---|---|---|
| Composé de l'exemple n° | DE 50 et limites à 95 % (mg/kg/I.P.) | Puissance relative | |
| | | Cimétidine = 1 | Ranitidine = 1 |
| 1 | 19 (9-40) | 2,2 | 0,75 |
| 3 | 8 (4,4 -14) | 5,25 | 1,75 |
| 6 | > 50 | | |
| 8 | 18 (7,5-43) | 2,3 | 0,8 |
| 11 | 8 (4-16) | 5,2 | 1,75 |
| 13 | 1,5 (0,9-2,4) | 28 | 9,3 |
| 18 | 15 (8,3-27) | 2,8 | 0,9 |
| 19 | > 50 | | |
| 20 | 20 (9,5-42) | 2,1 | 0,7 |
| 21 | 32 (18-58) | 1,3 | 0,4 |
| 22 | 0,5 (0,3-0,8) | 84 | 28 |
| 28 | 1 (0,3-3) | 42 | 14 |
| 29 | 0,9 (0,3-2,5) | 46,7 | 15,5 |
| 30 | 0,5 (0,2-1,2) | 84 | 28 |
| 31 | 3,6 (1,9-6,8) | 11,7 | 3,9 |
| 33 | 0,7 (0,3-1,6) | 60 | 20 |
| 34 | 11 (6 -21) | 3,8 | 1,3 |
| Ranitidine | 14 (6-32,2) | 3 | 1 |
| Cimétidine | 42 (24,7-71,4) | 1 | 0,3 |
| Composé A | > 50 | | |

TABLEAU 2

| Oreillette isolée de cobaye | | | |
|---|---|---|---|
| Composé de l'exemple n° | pA2 et limites à 95 % | Puissance relative: Cimétidine = 1 | Puissance relative: Ranitidine = 1 |
| 1 | 6,98 (6,70-7,27) | 1,10 | 0,35 |
| 3 | 7,11 (6,85-7,38) | 1,48 | 0,47 |
| 6 | 6,21 (5,97-6,46) | 0,19 | 0,06 |
| 8 | 6,89 (6,66-7,13) | 0,89 | 0,28 |
| 11 | 6,85 (6,64-7,06) | 0,81 | 0,26 |
| 13 | 7,68 (7,46-7,90) | 5,50 | 1,74 |
| 18 | *7,05 (6,82-7,29) | 1,29 | 0,41 |
| 19 | 5,90 (5,67-6,14) | 0,09 | 0,03 |
| 20 | 7,37 (7,17-7,58) | 2,69 | 0,85 |
| 21 | 7,10 (6,87-7,34) | 1,45 | 0,46 |
| 22 | *7,77 (7,53-8,02) | 6,76 | 2,14 |
| 28 | *7,37 (7,08-7,67) | 2,69 | 0,85 |
| 29 | *7,55 (7,29-7,81) | 4,07 | 1,29 |
| 30 | *7,19 (6,95-7,44) | 1,78 | 0,56 |
| 31 | *7,51 (7,26-7,77) | 3,72 | 1,17 |
| 33 | *7,31 (7,01-7,62) | 2,34 | 0,74 |
| 34 | 7,20 (6,94-7,47) | 1,82 | 0,58 |
| Ranitidine | 7,44 (7,12-7,76) | 3,16 | 1 |
| Cimétidine | 6,94 (6,77-7,11) | 1 | 0,32 |
| Composé A | 5,88 (5,66-6,1 ) | 0,087 | 0,028 |

*inhibition difficilement réversible

Tableau 3

| Hyperacidité gastrique à l'histamine chez le rat anesthésié | | | |
|---|---|---|---|
| Composé de l'exemple n° | DE 50 et limites à 95 % (mg/kg/I.V.) | Puissance relative | |
| | | Cimétidine = 1 | Ranitidine = 1 |
| 1 | 0,6 (0,25-1,5) | 0,7 | 0,15 |
| 3 | 0,3 (0,1 -0,9) | 1,3 | 0,3 |
| 6 | > 5 | | |
| 8 | 0,5 (0,18-1,4) | 0,8 | 0,18 |
| 11 | 0,4 (0,15-1) | 1 | 0,22 |
| 13 | 0,03 (0,02-0,05) | 13,3 | 3 |
| 18 | 0,5 (0,17-1,5) | 0,8 | 0,18 |
| 19 | > 5 | | |
| 20 | 0,1 (0,03-0,3) | 4 | 0,9 |
| 21 | 0,6 (0,3 -1,2) | 0,7 | 0,15 |
| 22 | 0,05 (0,02-0,11) | 8 | 1,8 |
| 28 | 0,2 (0,08-0,5) | 2 | 0,45 |
| 29 | 0,1 (0,04-0,25) | 4 | 0,9 |
| 30 | 0,2 (0,07-0,6) | 2 | 0,45 |
| 31 | 0,15 (0,07-0,3) | 2,7 | 0,6 |
| 33 | 0,2 (0,08-0,5) | 2 | 0,45 |
| 34 | 0,25 (0,12-0,5) | 1,6 | 0,36 |
| Ranitidine | 0,09 (0,05-0,17) | 4,4 | 1 |
| Cimétidine | 0,4 (0,18-0,9) | 1 | 0,22 |
| Compose A | > 5 | | |

Tableau 4

| Hyperacidité gastrique à l'histamine chez le rat anesthésié | | | | |
|---|---|---|---|---|
| Composé de l'exemple n° | n* | Durée d'action (minute ± E.S.) | Puissance relative Cimétidine = 1 | Ranitidine = 1 |
| 1 | 3 | 112 ± 27 | 1,6 | 1,4 |
| 3 | 4 | 90 ± 20 | 1,3 | 1,2 |
| 8 | 3 | 128 ± 22 | 1,8 | 1,7 |
| 11 | 3 | 88 ± 24 | 1,2 | 1,1 |
| 13 | 5 | 120 ± 18 | 1,7 | 1,6 |
| 18 | 3 | > 8 heures | > 6,2 | > 6,7 |
| 20 | 3 | 102 ± 22 | 1,4 | 1,3 |
| 21 | 3 | 76 ± 20 | 1,1 | 1 |
| 22 | 8 | > 8 heures | > 6,2 | > 6,7 |
| 28 | 4 | > 8 heures | > 6,2 | > 6,7 |
| 29 | 4 | > 8 heures | > 6,2 | > 6,7 |
| 30 | 3 | > 8 heures | > 6,2 | > 6,7 |
| 31 | 3 | > 8 heures | > 6,2 | > 6,7 |
| 33 | 3 | > 8 heures | > 6,2 | > 6,7 |
| 34 | 3 | 85 ± 21 | 1,2 | 1,1 |
| Ranitidine | 5 | 71 ± 13 | | |
| Cimétidine | 7 | 77 ± 10 | | |

* n = nombre d'essais

La présente invention concerne les médicaments constitués par les produits de formules générale $_{II}$ pris tels que, éventuellement sous forme d'un sel, à l'état pur ou sous forme d'associations avec tout autre produit acceptable du point de vue pharmaceutique lequel peut être inerte ou physiologiquement actif. Ces médicaments peuvent être administrés selon une grande variété de formes posologiques différentes telles que comprimés, gélules, capsules, poudres, granulés, etc... Dans ces compositions, le principe actif est mélangé à un ou plusieurs diluants inertes tels que lactose ou amidon, en outre ces compositions peuvent comprendre des substances autres que des diluants par exemple des lubrifiants tels que talc ou stéarate de magnésium ; lorsqu'on désire des suspensions aqueuses, élixirs ou sirops pour administration orale, l'ingrédient actif essentiel peut y être associé à divers agents édulcorants, et/ou aromatisants, le cas échéant des émulsionnants et/ou des agents de mise en suspension en même temps que des diluants tels que l'eau, l'éthanol, le propylène glycol et diverses associations similaires.

Une composition pharmaceutique se prêtant à l'administration par voie orale et présentée sous forme de dose unitaire contenant 5 à 500 mg de principe actif est considérée comme intéressante.

L'exemple suivant, donné à titre non limitatif, illustre une composition de ce type.

<u>Exemple</u> :

| | |
|---|---|
| Principe actif ..................... | 10 mg |
| Lactose ........................... | 100 mg |
| Amidon de blé ....................... | 30 mg |
| Talc .............................. | 6 mg |
| Polyvidone excipient ............... | 3 mg |
| Stéarate de magnésium .............. | 1 mg |

Pour préparer les composés de la formule générale II dans laquelle $X_1$ et $X_2$ représentent tous deux un atome de soufre et U un reste diamino-3,4 cyclobutènedione-1,2, on fait réagir successivement, dans les conditions décrites par S. Cohen et S.G. Cohen, J. Amer. Chem. Soc., 1966, 88, 1533-1536 une dialkoxy-3,4 cyclobutènedione-1,2, dont la préparation est également décrite dans cette publication, avec une amine de formule générale VI.

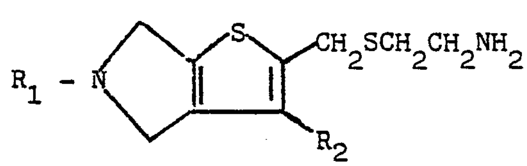

(VI)

dans laquelle $R_1$ et $R_2$ ont les mêmes significations que précedemment, puis le composé intermédiaire ainsi obtenu avec l'ammoniac.

Les composés de la formule générale II, dans laquelle $X_1$ et $X_2$ représentent un atome de soufre et U un reste N-aminosulfonylamidine, peuvent être préparés par réaction du sulfamide sur un iminoéther de formule générale VII.

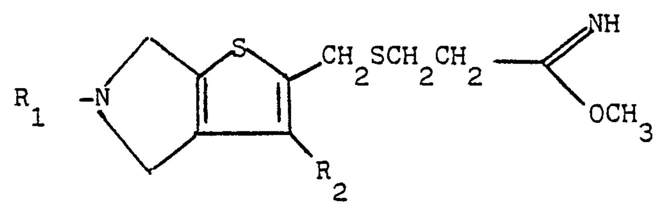

(VII)

dans laquelle $R_1$ et $R_2$ ont les mêmes significations que précedemment.
La réaction est effectuée au sein d'un solvant polaire de préférence un alcool tel que le méthanol, sous atmosphère inerte et à une température voisine de la température ambiante.

Les composés de la formule générale II dans laquelle m a la valeur 2 et U désigne un reste azoté répondant à la formule III, peuvent être préparés par la réaction d'un composé de formule générale VIII,

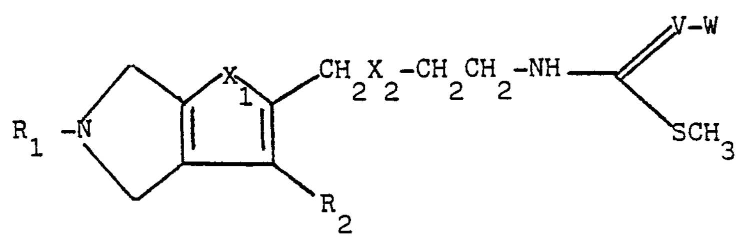

(VIII)

dans laquelle $X_1$, $X_2$, $R_1$, $R_2$, V et W, ont les mêmes significations que précédemment, avec un composé aminé de formule $R_3NH_2$, dans laquelle $R_3$ possède les significations précédemment définies ; la réaction peut être

exécutée dans tout solvant ou diluant compatible avec les composés en présence, mais de préférence dans un solvant assez polaire tel que l'éthanol, le méthanol ou l'acétonitrile et à une température pouvant être comprise entre la température ambiante et la température de reflux du solvant utilisé.

Un deuxième procédé pour préparer les composés de la formule II dans laquelle m a la valeur 2, U désigne un reste azoté de formule III et $X_1$ représente un atome de soufre, consiste à faire réagir un composé halogéné de formule IX ou l'un de ses sels, de préférence le chlorhydrate,

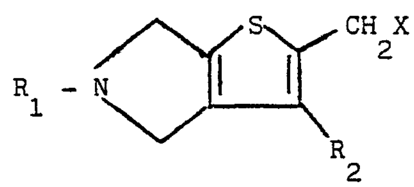

(IX)

dans laquelle X désigne un halogène de préférence le chlore, $R_1$ et $R_2$ ayant les mêmes significations que précédemment, avec un thiol de formule générale X.

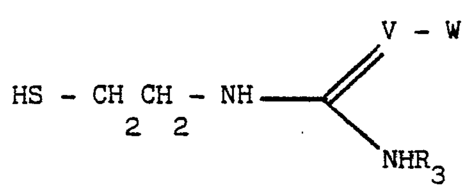

(X)

dans laquelle $R_3$ a les significations précédemment définies. La réaction est exécutée dans un solvant hydroxylé comme un alcool, à température ambiante et en présence d'un agent basique qui peut être un hydrure, un alcoolate, un carbonate ou un bicarbonate alcalin mais de préférence un alcoolate de sodium, préparé "in situ", lorsque c'est un alcool qui est choisi comme solvant.

Les thiols de formules X peuvent être préparés à partir d'un composé de formule XI

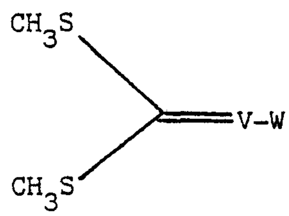

(XI)

dans laquelle V et W ont les mêmes significations que précédemment, que l'on fait réagir avec une amine de formule $R_3NH_2$, dans laquelle $R_3$ a les significations précédentes, dans les conditions décrite par J.S. Davidson, Chem. Ind. (London), 1965, 48, 1964-5, et/ou dans le cas où l'enchaînement = V-W représente un groupement -NCN, selon le procédé de F.H.S. Curd et Coll., J. Chem. Soc., 1948, 1630-6, à partir du cyanamide de sodium et d'un isothiocyanate de formule $R_3NCS$ ; puis le composé ainsi obtenu est traité par le chlorhydrate de cystéamine en milieu hydroalcoolique en présence d'un agent basique comme la soude.

Les composés de formule générale XI, dans laquelle le motif =V-W représente un groupement = N-CN ou = $CH-NO_2$ sont connus et leur préparation décrite respectivement par R.J. Timmons et L.S. Wittenbrook, J. Org., Chem., 1967, 32, 1566-72 et par R. Gompper et H. Schaefer, Chem. Ber., 1967, 100, 591-604.

Pour préparer les composés de la formule générale II, dans laquelle m est égal à 2 et U représente un reste aminotriazole de formule générale IV, on fait réagir un composé de formule générale VIII dans laquelle le motif =V-W désigne un enchaînement =NCN, avec une alkylhydrazine de formule $R_4NHNH_2$, dans laquelle $R_4$ a les significations précédentes ; la réaction peut être effectuée dans tout solvant inerte vis à vis des composés en présence comme, par exemple, un hydrocarbure aromatique tel que le toluène, le xylène, le triméthylbenzène, etc... et à une température qui peut être comprise entre la température ambiante et la température de reflux du solvant utilisé.

Les composés de la formule II dans laquelle U représente un reste aminothiadiazole de formule V peuvent être obtenus en faisant réagir une amine de formule générale XII,

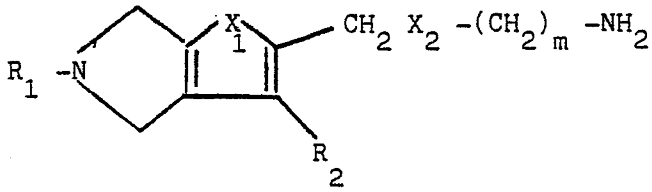

(XII)

successivement, avec un dialkoxy-3,4 thiadiazole [1,2,5] S-oxyde et une amine de formule $R_3NH_2$ dans laquelle $R_3$ a les significations précédentes, selon les indications de S. Karady et Coll., Heterocycles, 1981, 16, 1561.

Un autre procédé pour la préparation des composés de la formule générale II dans laquelle U désigne un reste thiadiazole de formule V et $X_1$ et $X_2$ représentent tous deux un atome de soufre consiste à faire réagir un alcool de formule XIII.

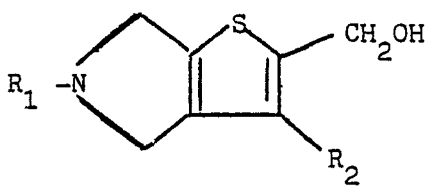

(XIII)

dans laquelle $R_1$ et $R_2$ ont les mêmes significations que précédemment, avec un thiol de formule XIV dans laquelle, $R_3$ et m ont les significations précédemment indiquées et qui peut

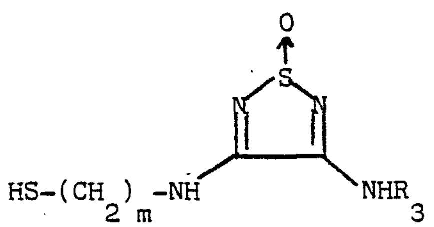

(XIV)

être préparé dans les conditions de la publication de S. Karady et Coll., citée ci-dessus, en faisant réagir sur un dialkoxy-3,4 thiadiazole [1,2,5] S-oxyde, successivement la cystéamine et une amine de formule $R_3NH_2$ ; la réaction du composé XIII avec le composé XIV est effectuée en présence d'un agent de déshydratation de préférence l'acide chlorhydrique concentré et à une température voisine de 0°C.

Les dialkoxy-3,4 thiadiazoles [1,2,5] -S-oxydes utilisés sont des composés connus, qui ont été préparés d'après les indications de S. Karady et Coll., Heterocycles, 1981, 16, 1561.

Les amines intermédiaires, de formule générale VI définie précédemment sont des composés nouveaux qui peuvent être préparés comme le montre le schéma réactionnel 1 suivant,

Schéma 1

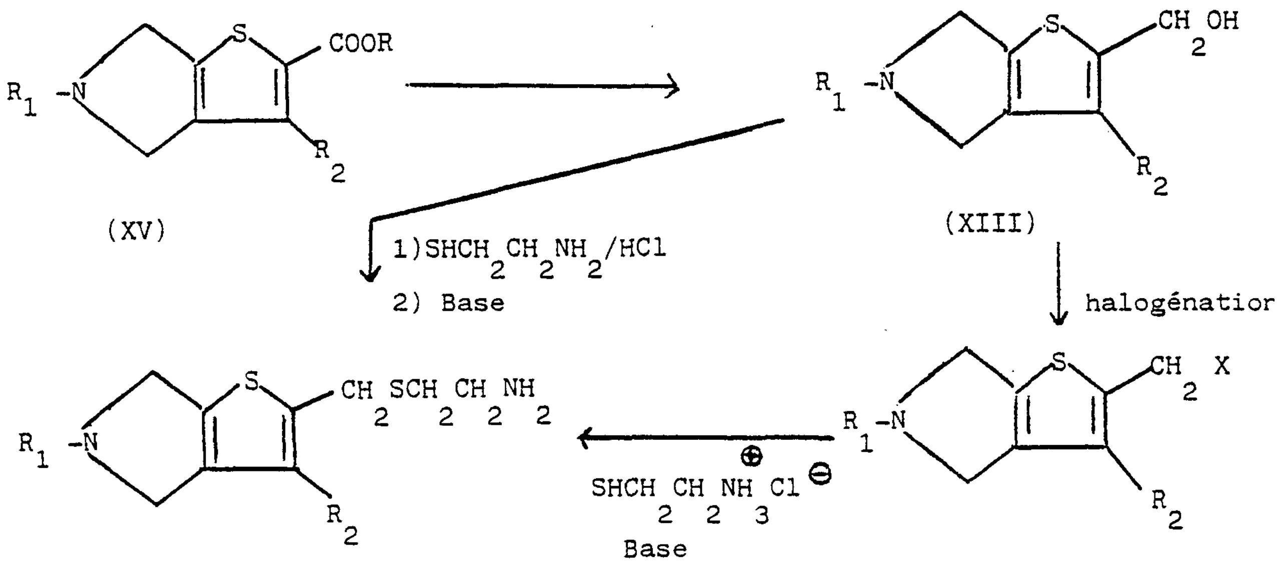


(XV) (XIII)

(VI) (IX)

selon lequel on réduit un ester de formule générale XV, dans laquelle R désigne un groupement alkyle de préférence méthyle ou éthyle en l'alcool correspondant XIII à l'aide des réducteurs de la fonction ester tels que le diborane ou l'hydrure de lithium et d'aluminium au sein d'un solvant approprié comme l'éther ou le tétrahydrofuranne. Lorsqu'on met à réagir les alcools XIII ainsi obtenus avec la cystéamine en présence d'un agent déshydratant dans les conditions déjà indiquées (cf. condensation d'un alcool XIII avec un thiol XIV) on obtient avec un rendement acceptable les amines VI souhaitées.

Un deuxième procédé consiste à transformer un alcool XIII en l'halogénure correspondant IX lequel est ensuite traité par la cystéamine en milieu basique dans les conditions déjà mentionnées, (cf. alkylation des thiols de formule X) ; l'halogénation des alcools XIII peut être effectuée au moyen des réactifs usuels comme le chlorure de thionyle au sein d'un solvant inerte tel que le chloroforme ou le chlorure de méthylène et à une température comprise entre 0°C et la température de reflux mais de préférence à une temprérarture voisine de la température ambiante.

Les iminoéthers de formule générale VII sont obtenus par action du méthanol sur un nitrile de formule générale XVII.

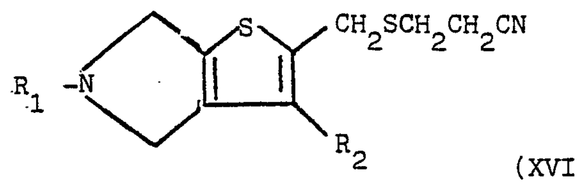

(XVII)

dans laquelle $R_1$ et $R_2$ ont les significations précédentes, dans les conditions habituelles de préparation des iminoéthers telles qu'on peut les trouver décrites par exemple dans Houben-Weyl, Methoden der Organischen Chemie, 1952, 8, 697.

Les composés de formule générale XVII sont nouveaux et peuvent être préparés selon la séquence réactionnelle du schéma 2 suivant,

Schéma 2

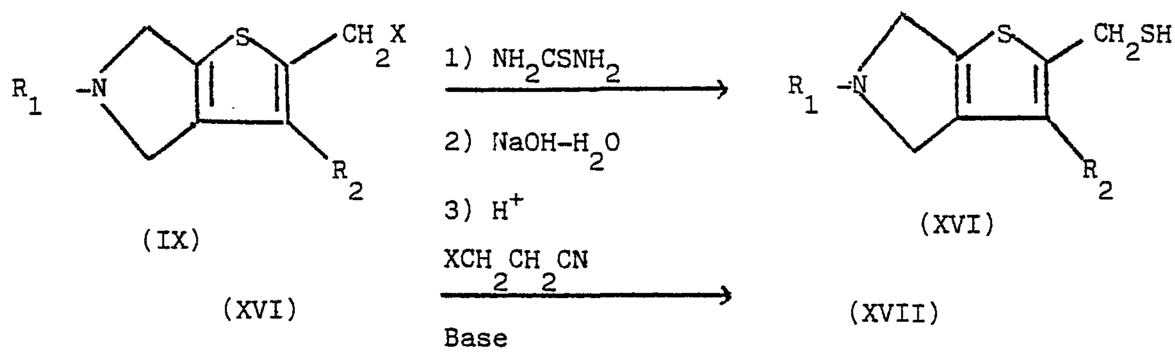


(IX) (XVI) (XVI) (XVII)

par alkylation d'un halogénure IX par la thiourée puis hydrolyse du sel d'isothiouronium intermédiaire dans les conditions habituelles telles que décrites notamment dans Houben-Weyl, Methoden der Organischen Chemie, 1955, 9, 14, de façon à obtenir le thiol correspondant de formule XVI lequel peut être ensuite alkylé par un propane nitrile ω-halogéné de préférence le chloro-3 propane nitrile ; Cette alkylation est effectuée dans un solvant polaire comme le méthanol en présence d'un agent basique tel que le méthylate de sodium et à une température comprise entre 0°C et la température ambiante.

Les composés de formule générale VIII sont préparés en faisant réagir une amine de formule générale XII, dans laquelle m est égal à 2, avec un composé de formule XI au sein de tout solvant ou diluant compatible avec les réactifs en présence, mais de préférence dans un solvant assez polaire comme l'acétonitrile et à une température qui est le plus souvent la température de reflux du solvant choisi.

Les amines de formule générale XII, dans laquelle $X_1$ désigne un atome de soufre, $X_2$ est un atome d'oxygène et m est égal à 2 peuvent être préparées par condensation d'un halogènure de formule IX avec l'éthanolamine, la réaction étant exécutée de préférence au sein d'un excès d'éthanolamine.

Les amines de formule générale XII, dans laquelle $X_1$ et $X_2$ désignent tous deux un atome de soufre et m égale 3 peuvent être synthétisées par alkylation d'un thiol de formule XVI par une ω-halogéno propylamine de préférence la bromo-3 propylamine en milieu basique ou bien par réduction d'un nitrile de formule générale XVII au moyen d'un réducteur connu de la fonction nitrile comme par exemple l'hydrure de lithium et d'aluminium dans un solvant adapté comme l'éther ou le tétrahydrofuranne.

Les amines de formule générale XII dans laquelle $X_1$ désigne un atome d'oxygène, $X_2$ un atome de soufre et m égale 2 peuvent être préparés par réduction d'un ester de formule générale XIX à l'aide d'un réducteur de la fonction ester dans le solvant approprié puis condensation de l'alcool XVIII ainsi obtenu avec la cystéamine comme illustré sur le schéma 3 suivant,

Schéma 3

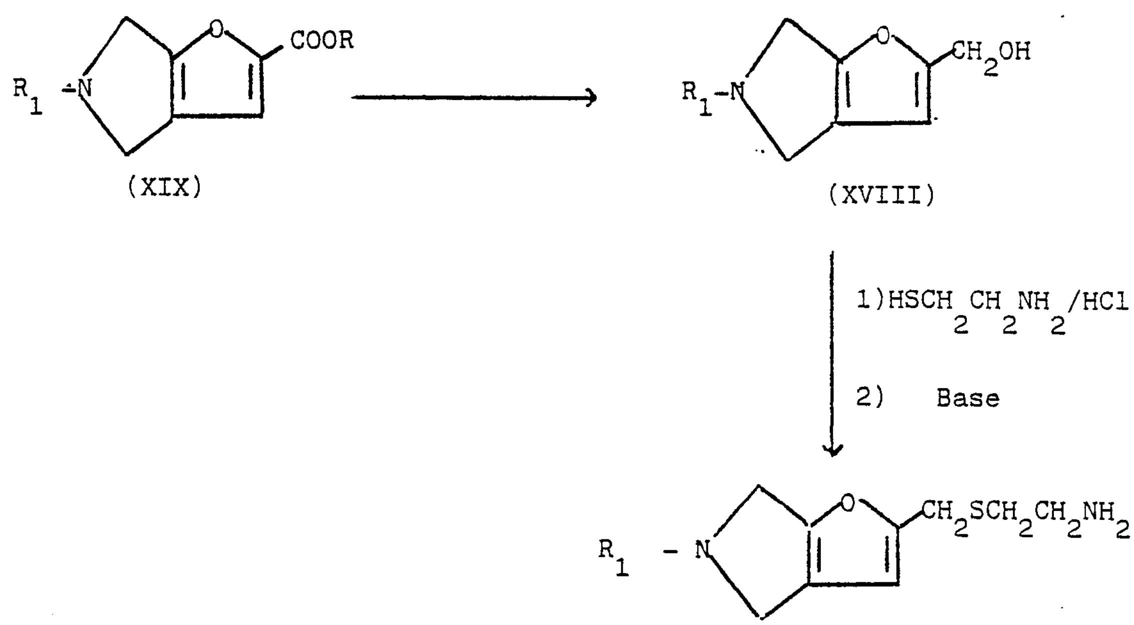


(XIX)

(XVIII)

Les thiénopyrroles carboxylates d'alkyle de formule générale XV sont pour certains d'entre eux déjà décrits ; ces composés peuvent être préparés selon la séquence réactionnelle connue, suivante,

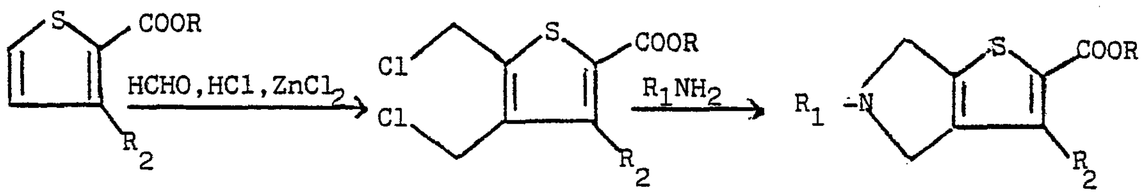


(XXI) (XX) (XV)

en partant d'un ester XXI que l'on soumet à une réaction de chlorométhylation, on obtient ainsi le dihalogénure XX lequel est ensuite cyclisé en l'ester XV souhaité par réaction avec une amine $R_1NH_2$, selon la technique de J. Feijen et H. Wynberg Rec. Trav. Chim. Pays Bas, 1970, <u>89</u>, 639.

Comme agent de chlorométhylation on peut utiliser le mélange polyoxyméthylène-acide chlorhydrique tel que décrit par Cho Sone, Nippon Kagaku Zasshi, 1965, <u>86</u>, 1331 - C.A., <u>65</u>, 13637c, de préférence au chlorométhyl méthyl éther réputé toxique.

Les esters de formule générale XIX qui sont des produits nouveaux, peuvent être préparés à partir du méthyl-5 furanne-2 carboxylate de méthyle comme illustré sur le schéma 4 suivant,

<u>Schéma 4</u>

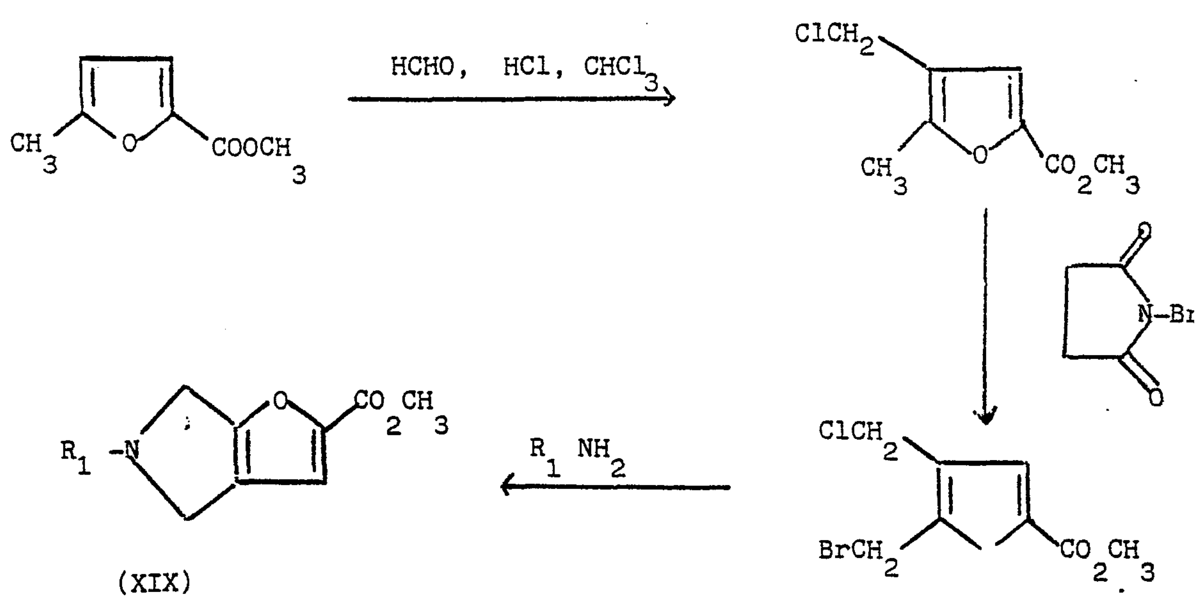


(XIX)

Sur ce schéma, les chlorométhyl-3 méthyl-2 furanne-5 carboxylate de méthyle et bromométhyl-2 chlorométhyl-3 furanne-5 carboxylate de méthyle sont des produits connus et décrits respectivement par A.L. Mndzhoyan et Coll., Doklady Akad. Nauk. Armyan, 1957, 25, 277 -CA 52, 12835 b, et M. Valenta et Coll., Collection Czech. Chem. Commun., 1964, 29, 1577 - C.A., 61, 6976 g ; la réaction de cyclisation avec une amine de formule $R_1NH_2$ est effectuée de préférence dans un solvant polaire aprotique comme l'acétonitrile, sous atmosphère inerte et à une température voisine de la température ambiante.

Les amines représentées par la formule,

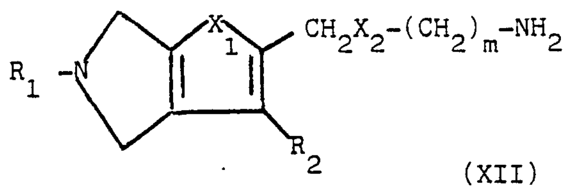

(XII)

dans laquelle $R_1$, $R_2$, $X_1$, $X_2$ et m ont les significations précédemment définies avec la condition que lorsque $X_1$ représente un atome d'oxygène, $R_2$ représente un atome d'hydrogène, $X_2$ un atome de soufre et m égale 2, intermédiaires dans la préparation de composés selon la formule II font partie de l'invention.

De même, les composés de formule XXII

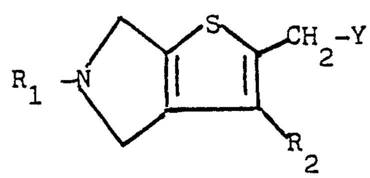

(XXII)

dans laquelle, $R_1$ et $R_2$ ont les significations précédemment définies et Y désigne les groupements : OH, halogène, SH, $-S(CH_2)_2CN$, $-S(CH_2)_2$ 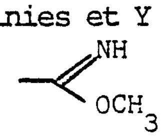 ,

sont des intermédiaires dans la préparation des composés de formule II quand $X_1$ est un atome de soufre, faisant partie de l'invention.

En outre, les composés de formule XXIII,

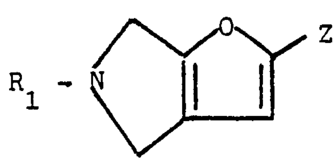

(XXIII)

dans laquelle $R_1$ a les significations énoncées précédemment et Z désigne un groupement : $-COOCH_3$ ou $-CH_2OH$, sont des intermédiaires dans la préparation des composés de formule II quand $X_1$ représente un atome d'oxygène et, à ce titre, font partie de l'invention.

Il est donné ci-après des exemples illustrant l'invention de manière non limitative. Les points de fusion sont indiqués en degrés centigrades et les points d'ébullition en millibars.

EXEMPLE 1 : N-Cyano N'-[ [ (éthyl-5 dihydro-5,6-4H-thiéno [ 2,3-c ] pyrrolyl-2) méthylthio ]-2 éthyl ]N"-méthyl guanidine (Formule 1)

A une solution de méthylate de sodium dans le méthanol, préparée par dissolution de 0,9 g (0,039 at.gr.) de sodium dans 50 $cm^3$ de méthanol, on ajoute à une température comprise entre 0 et 5°C, une solution de 5,6 g (0,035 mole) de N-cyano N'-(mercapto-2 éthyl) N"-méthyl guanidine dans 40 $cm^3$ de méthanol ; on agite le mélange pendant 2 heures à 0°C puis on y rajoute à cette température, 4,2 g (0,0176 mole) de chlorhydrate du chlorométhyl-2 éthyl-5 dihydro-5,6-4H-thiéno [ 2,3-c ] pyrrole ; l'agitation est poursuivie pendant 42 heures à température ambiante, après quoi on évapore le mélange, dissout le résidu dans la quantité suffisante de chloroforme, on lave la solution ainsi obtenue à la soude puis à l'eau, on sèche la phase organique sur sulfate de sodium, on filtre, on évapore le solvant, disperse le résidu dans le diisopropyléther, essore et sèche. Poids = 3,3 g Rdt = 58 %

F = 129 - 132°C F. = 142 - 143°C (isopropanol)

R.M.N. (DMSO-$d_6$)

1,3 (t) 3H ($CH_3CH_2$) ; 2,6-3,3 (m) 7H ($CH_3-CH_2N$, $CH_3N$, $SCH_2$)
3,8-4,3 (m) 8H ($CH_2S$, $N-CH_2$) ; 6,8-7,3 (m) 3H (NH, H aromatique)

Analyse centésimale : $C_{14}H_{21}N_5S_2$ P.M. = 323,48

| | C | H | N | S |
|---|---|---|---|---|
| % calculé | 51,98 | 6,54 | 21,65 | 19,83 |
| % trouvé | 52,02 | 6,55 | 21,81 | 19,53 |

EXEMPLE 2 : N- [ [ (Ethyl-5 dihydro-5,6-4H-thiéno [ 2,3-c ] pyrrolyl-2) méthylthio ] -2 éthyl ] méthylthio-1 nitro-2 éthènamine (Formule 2)

On chauffe à reflux pendant 12 heures un mélange de 7,8 g (0,032 mole) d'/ (éthyl-5 dihydro-5,6-4H-thiéno [ 2,3-c ] pyrrolyl-2) méthylthio ]-2 éthylamine et 5,3 g (0,032 mole) de bis méthylthio-2,2' nitro-2 éthène, dans 100 $cm^3$ d'acétonitrile ; on filtre ensuite le mélange réactionnel, on évapore le filtrat à sec, on disperse le résidu ainsi obtenu dans la

quantité minimum d'acétate d'éthyle, on essore, lave le solide au diisopropyléther, puis on le sèche. Poids = 7 g Rdt = 61 %

F = 85 - 87°C F. = 93 - 4°C (diisopropyléther - isopropanol, 1-1)

R.M.N. ($CDCl_3$)

1,2 (t) 3H($CH_3$-$CH_2$) ; 1,8 (s) 1H (NH) ; 2,4 (s) 3H (CH3S) ;
2,6-3,1 (m) 4H ($SCH_2$, $CH_3CH_2N$) ; 3,33 - 4,1 (m) 8H ($CH_2N$, $CH_2S$)
6,6 (s) et 6,75 (s) 2H (=CH, H aromatique)

Analyse : $C_{14}$ $H_{21}$ $N_3$ $O_2$ $S_3$ P.M. = 359,53

| | C | H | N | S |
|---|---|---|---|---|
| % calculé | 46,77 | 5,89 | 11,69 | 26,75 |
| % trouvé | 46,52 | 5,90 | 11,85 | 26,45 |

EXEMPLE 3 : N-[ [ (Ethyl-5 dihydro-5,6-4H-thiéno [ 2,3-c ] pyrrolyl-2) méthylthio ]-2 éthyl ]N'-méthyl nitro-2 éthènediamine-1,1 (Formule 3)

On chauffe à reflux pendant 11 heures une solution de 5,3 g (0,0147 mole) du composé de l'exemple 2 dans 15 $cm^3$ d'une solution de méthylamine à 33% dans l'éthanol ; on évapore ensuite le mélange, on disperse le résidu obtenu dans la quantité suffisante d'acétate d'éthyle, on l'essore et on le sèche. Poids = 4,6 g Rdt = 92 % F = 96 - 101°C

F. = 99 - 101°C (acétate d'éthyle -isopropanol, 2-1)

R.M.N. ($CDCl_3$)

1,2 (t) 3H ($CH_3$-$CH_2$) ; 2,6-4,08 (m) 15H ($CH_2$) ; 6,6-7,3 (m)
3H (=CH,NH) ; 10,1 (s élargi) 1H (NH)

Analyse : $C_{14}$ $H_{22}$ $N_4$ $O_2$ $S_2$ P.M. = 342,48

| | C | H | N | S |
|---|---|---|---|---|
| % calculé | 49,09 | 6,48 | 16,36 | 18,73 |
| % trouvé | 49,09 | 6,39 | 16,19 | 18,90 |

EXEMPLE 4 : N-Cyano N'- [ [ (éthyl-5 dihydro-5,6-4H-thiéno [ 2,3-c ] pyrrolyl-2) méthylthio ]-2 éthyl ]N"-isopropyl guanidine (Formule 4)

A une solution méthanolique de méthylate de sodium, préparée par dissolution de 1 g (0,0434 at.gr.) de sodium dans 50 $cm^3$ de méthanol et refroidie entre 0 et 5°C, on ajoute 7,2 g (0,0386 mole) de N-cyano N'-isopropyl N"-(mercapto-2 éthyl) guanidine en solution dans 85 $cm^3$ de méthanol ; on agite le mélange pendant 2 heures entre 0 et 5°C puis on y ajoute 4,6 g (0,0193 mole) de chlorhydrate du chlorométhyl-2 dihydro-5,6 éthyl-5-4H-thiéno [ 2,3-c ] pyrrole en solution dans 50 $cm^3$ de méthanol ; on poursuit l'agitation du mélange pendant 24 heures à température ambiante, puis on évapore, on dissout le résidu obtenu dans la quantité

suffisante de chloroforme, on lave la solution ainsi obtenue à la soude puis à l'eau, on sèche la phase organique sur sulfate de sodium, on filtre, on évapore le solvant et on disperse le résidu dans l'acétate d'éthyle, on essore et on sèche : Poids = 6,8 g Rdt = 47 %

F = 92 - 95°C F. = 97 - 99°C (diisopropyléther-isopropanol, 5-1)

Analyse : $C_{16}H_{25}N_5S_2$ P.M. = 351,53

| | C | H | N | S |
|---|---|---|---|---|
| % calculé | 54,66 | 7,17 | 19,93 | 18,24 |
| % trouvé | 54,40 | 7,24 | 20,16 | 18,31 |

Les composés des exemples 5,6 et 7 suivants sont préparés selon le mode opératoire décrit à l'exemple 1 en utilisant le chlorométhyl-2 thiéno [2,3-c] pyrrole approprié.

EXEMPLE 5 : N-Cyano N' -[[(dihydro-5,6 méthyl-5-4H-thiéno [2,3-c] pyrrolyl-2) méthylthio]-2 éthyl]N"-méthyl guanidine (Formule 5)

F = 145 - 147°C (isopropanol)

Analyse : $C_{13}H_{19}N_5S_2$ P.M. = 309,45

| | C | H | N | S |
|---|---|---|---|---|
| % calculé | 50,46 | 6,19 | 22,63 | 20,72 |
| % trouvé | 50,34 | 6,21 | 22,45 | 20,57 |

EXEMPLE 6 : N-Cyano N' -[[(dihydro-5,6 isopropyl-5-4H-thiéno [2,3-c] pyrrolyl-2) méthylthio]-2 éthyl] N" méthyl guanidine (Formule 6)

F = 120 - 121°C (diisopropyléther - isopropanol, 2-1)

Analyse : $C_{15}H_{33}N_5S_2$ P.M. = 337,50

| | C | H | N | S |
|---|---|---|---|---|
| % calculé | 53,38 | 6,87 | 20,75 | 19,00 |
| % trouvé | 53,49 | 6,97 | 20,99 | 19,17 |

EXEMPLE 7 : N-[[(Benzyl-5 dihydro-5,6-4H-thiéno [2,3-c] pyrrolyl-2) méthylthio]-2 éthyl]N'-cyano N"-méthyl guanidine (Formule 7)

F = 122 - 123°C (diisopropyléther - isopropanol, 1-3)

Analyse : $C_{19}H_{23}N_5S_2$ P.M. = 385,54

| | C | H | N | S |
|---|---|---|---|---|
| % calculé | 59,19 | 6,01 | 18,17 | 16,63 |
| % trouvé | 59,02 | 6,10 | 17,90 | 16,58 |

EXEMPLE 8 : [(Ethyl-5 dihydro-5,6-4H-thiéno [2,3-c] pyrrolyl-2) méthylthio]-3 N-sulfamoyl propanamidine (Formule 8)

On agite, dans un réacteur placé sous atmosphère d'azote, pendant 48 heures, un mélange de 11,3 g (0,0397 mole) d' [(éthyl-5

dihydro-5,6-4H-thiéno [ 2,3-c ] pyrrolyl-2) méthylthio ]-3 propanimidate de méthyle et 7,6 g (0,08 mole) de sulfamide dans 100 $cm^3$ de méthanol ; on évapore, on reprend le résidu par du chloroforme, on filtre, on évapore le filtrat et on cristallise le résidu dans l'isopropanol.

Poids = 3,8 g   Rdt = 28 %   F = 124 - 130°C

On dissout ce solide dans le chloroforme en y ajoutant un peu de méthanol et on chromatographie cette solution sur colonne de gel de silice en utilisant une solution à 5 % de méthanol dans le chloroforme comme éluant. Poids = 1,1 g   F = 137 - 139°C (isopropanol - éthanol, 4-1)

R.M.N. (DMSO-$d_6$)

1 (t) 3H ($CH_3$) ; 2,25-3 (m) 6H ($CH_2$-C, $SCH_2$, $CH_2N$) ;
3-4,2 (m) 6H ($CH_2S$, $NCH_2$) ; 6,4 (s) 2H ($NH_2$) ;
6,75 (s) 1H (H thiophène) ; 7-8,2 (m) 2H ($NH_2$)

Analyse : $C_{12}$ $H_{20}$ $N_4$ $O_2$ $S_3$   P.M. = 348,51

| | C | H | N | S |
|---|---|---|---|---|
| % calculé | 41,35 | 5,79 | 16,08 | 27,60 |
| % trouvé | 41,58 | 5,71 | 16,03 | 27,40 |

EXEMPLE 9 : N-Cyano N'-[ [ (éthyl-5 dihydro-5,6-4H-thiéno [ 2,3-c ] pyrrolyl-2) méthylthio ]-2 éthyl ]N"-éthylguanidine (Formule 9)

On chauffe à reflux pendant 5 h 30 une solution de 5 g (0,0147 mole) de N-cyano N'-[ [ (éthyl-5 dihydro-5,6-4H-thiéno [ 2,3-c ] pyrrolyl-2) méthylthio ]-2 éthyl ] S-méthyl isothiourée et 19,9 g (0,441 mole) d'éthylamine dans 30 $cm^3$ d'éthanol ; on évapore ensuite la solution et on cristallise le résidu obtenu dans la quantité suffisante d'acétate d'éthyle, on essore et on sèche. Poids = 3,4 g  Rdt = 68 %   F = 91 - 3°C

On recristallise ce solide dans un mélange de 20 $cm^3$ de diisopropyléther et 10 $cm^3$ d'isopropanol puis dans un mélange de 15 $cm^3$ de diisopropyléther et 20 $cm^3$ d'acétate d'éthyle : Poids = 2,4 g

F = 95 - 6°C

Analyse : $C_{15}$ $H_{23}$ $N_5$ $S_2$   P.M. = 337,50

| | C | H | N | S |
|---|---|---|---|---|
| % calculé | 53,38 | 6,87 | 20,75 | 19,00 |
| % trouvé | 53,55 | 6,60 | 20,95 | 18,92 |

EXEMPLE 10 : N-Cyano N' -[ [ (éthyl-5 dihydro-5,6-4H-thiéno [ 2,3-c ] pyrrolyl-2) méthylthio ]-2 éthyl ]-N"-propargyl guanidine (Formule 10)

On chauffe à reflux pendant 8 heures un mélange de 3 g (0,0088 mole) de N-cyano N'-[ [ (éthyl-5 dihydro-5,6-4H-thiéno [ 2,3-c ] pyrrolyl-2)

méthylthio ]-2 éthyl ] S-méthyl isothiourée et 7,3 g (0,132 mole) de propargylamine dans 30 $cm^3$ de méthanol ; on évapore ensuite la solution puis on chromatographie le résidu en solution dans le chloroforme sur colonne de gel de silice en utilisant comme éluants successivement : chloroforme, acétate d'éthyle, chloroforme à 5 % de méthanol ; les fractions éluées à l'acétate d'éthyle et au chloroforme - méthanol sont réunies et évaporées à sec ; on obtient ainsi un solide : Poids = 1,7 g Rdt = 56 % F = 107 - 110°C F = 112 - 114°C (diisopropyléther - acétate d'éthyle, 1-3)

R.M.N. ($CDCl_3$)

1,2 (t) 3H ($CH_3$) ; 2,4 (m) 1H ( C-H) ;

2,6-3,3 (m) 4H ($S-CH_2$, $CH_2-C\equiv$) ; 3,3-3,6 (m) 2H ($CH_2N$) ;

3,6-4,2 (m) 8H ($NCH_2$, $CH_2S$) ; 5,8-6,6 (m) 2H (NH) ;

6,7 (s) 1H (H thiophène)

Analyse : $C_{16}H_{21}N_5S_2$ P.M. = 347,50

| | C | H | N | S |
|---|---|---|---|---|
| % calculé | 55,3 | 6,09 | 20,16 | 18,45 |
| % trouvé | 55,35 | 6,15 | 20,12 | 18,17 |

EXEMPLE 11 : N-[ [ (Ethyl-5 dihydro-5,6-4H-thiéno [2,3-c ] pyrrolyl-2) méthylthio ]-2 éthyl ] méthyl-1-1H-triazole [ 1,2,4 ] diamine-3,5 (formule 11)

On chauffe à reflux, sous atmosphère d'azote pendant 2 heures une solution de 4,25 g (0,0925 mole) de méthylhydrazine et 6,75 g (0,0925 mole) de N,N-diméthylformamide sec dans 60 cm³ de toluène ; on refroidit ensuite la solution à 30°C et on y ajoute 6,3 g (0,0185 mole) de N-cyano N'-[ [ (éthyl-5 dihydro-5,6-4H-thiéno [2,3-c ] pyrrolyl-2) méthylthio ] -2 éthyl ] S-méthyl isothiourée ; on chauffe à reflux le mélange pendant 7 heures puis on évapore le solvant, on lave le résidu à l'eau, on extrait au chloroforme, on sèche la phase chloroformique sur sulfate de sodium, on concentre et on chromatographie sur colonne de gel de silice en utilisant comme éluants successivement le chloroforme, l'acétate d'éthyle et une solution de chloroforme à 5 % dans le méthanol. La fraction éluée avec ce dernier mélange fournit, après évaporation et cristallisation du résidu dans du diisopropyléther additionné de quelques gouttes d'acétone, un solide qu'on recristallise dans l'acétate d'éthyle : Poids = 1,8 g Rdt = 28 % F = 109 - 112°C (diisopropyléther - acétate d'éthyle)

R.M.N. ($CDCl_3$)

1,08 (t) 3H ($\underline{CH_3}-CH_2$) ; 2,4-3,1 (m) 4H ; 3,1-4,6 (m) 14H ; 6,7 (s) 1H (H thiophène)

Analyse : $C_{14}H_{22}N_6S_2$ P.M. = 338,5

| | C | H | N | S |
|---|---|---|---|---|
| % calculé | 49,67 | 6,55 | 24,83 | 18,95 |
| % trouvé | 49,62 | 6,52 | 24,50 | 18,81 |

EXEMPLE 12 : N-cyano N'-[ [ (dihydro-5,6 phényl-5-4H-thiéno [ 2,3-c ] pyrrolyl-2) méthylthio ]-2 éthyl ] N"-méthyl guanidine (formule 12)

On chauffe à reflux un mélange de 2,5 g (0,0064 mole) de N-cyano N'-[ [ (dihydro-5,6 phényl-5-4H-thiéno [ 2,3-c ] pyrrolyl-2) méthylthio -2 éthyl ]-S-méthyl isothiourée, 30 cm³ d'une solution de méthylamine à 33 % dans l'éthanol et 30 cm³ de DMF et on y fait barboter un courant de méthylamine pendant 5 heures ; on évapore ensuite les solvants, on disperse le résidu dans l'isopropanol et on le recristallise dans la quantité suffisante d'un mélange eau-DMF (2-3) : Poids = 1,4 g F = 164 - 168°C Rdt = 58 %

R.M.N. (DMSO $D_6$)

2,85-3 (m) 5H ($CH_2S$, $CH_3N$) ; 3-3,6 (m) 2H ($CH_2NH$) ;
3,8-4,2 (s) 2H ($CH_2S$) ; 4,2-4,8 (m) 4H ($CH_2N$) ;
6,4-7,4 (m) 8H (H aromatique, NH)

Analyse : $C_{18}H_{21}N_5S_2$ P.M. = 371,52

| | C | H | N | S |
|---|---|---|---|---|
| % calculé | 58,19 | 5,70 | 18,85 | 17,26 |
| % trouvé | 58,19 | 5,73 | 19,01 | 17,18 |

EXEMPLE 13 : N-[ [ (Ethyl-5 dihydro-5,6-4H-thiéno [ 2,3-c ] pyrrolyl-2) méthylthio ]-2 éthyl ] oxyde-1 thiadiazole [ 1,2,5 ] diamine-3,4 (formule 13)

A une solution de 1,7 g (0,0089 mole) de diéthoxy-3,4 thiadiazole [ 1,2,5 ] oxyde-1 dans 20 cm³ de méthanol, on ajoute entre 5 et 10°, une solution de 2,15 g (0,0089 mole) d'[ (éthyl-5 dihydro-5,6-4H-thiéno [ 2,3-c ] pyrrolyl-2) méthylthio ]-2 éthylamine dans 20 cm³ de méthanol. On agite le mélange pendant 5 h 30 à température ambiante puis on y fait barboter pendant 10 mn à 0° un courant d'ammoniac. On poursuit l'agitation pendant 4 h à température ambiante puis on essore le solide obtenu : Poids = 1,4 g, F = 174°C (dec), Rdt = 60 %, F: 175°C (dec) (MeOH-DMF, 3-1)

Analyse : $C_{13} H_{19} N_5 OS_3$ P.M. = 357,52

| | C | H | N | S |
|---|---|---|---|---|
| % calculé | 43,67 | 5,36 | 19,59 | 26,91 |
| % trouvé | 43,60 | 5,35 | 19,62 | 26,73 |

EXEMPLE 14 : N-[ [ (dihydro-5,6 méthyl-5-4H-thiéno [ 2,3-c ] pyrrolyl-2) méthylthio ]-2 éthyl ]N'-méthyl nitro-2 éthène diamine-1,1 (formule 14)

On chauffe à reflux pendant 11 h 30 une solution de 2,4 g (0,0105 mole) de bis méthylthio-2,2' nitro éthène et de 1,75 g (0,0076 mole) de [(dihydro-5,6 méthyl-5-4H-thiéno [ 2,3-c ] pyrrolyl-2) méthylthio ]-2 éthylamine dans 30 cm³ d'acétonitrile puis on refroidit à -5°, on essore le solide obtenu (poids = 2,4 g, F = 112 - 114°C) et on le met en suspension dans 15 cm³ d'une solution de méthylamine à 33 % dans l'éthanol ; on chauffe ce mélange à reflux pendant 6 h 30 puis on évapore les solvants ; on isole ainsi un solide : Poids = 1,9 g, Rdt = 72 %, F = 108 - 112°C (acétate d'éthyle-isopropanol, 5-1)

Analyse : $C_{13}H_{19}N_3O_2S_3$ P.M. = 345,5

| | C | H | N | S |
|---|---|---|---|---|
| % calculé | 47,54 | 6,14 | 17,06 | 19,52 |
| % trouvé | 47,65 | 5,98 | 16,89 | 19,78 |

EXEMPLE 15 : N-[ [ (Ethyl-5 dihydro-5,6-4H-furo [ 2,3-c ] pyrrolyl-2) méthylthio ]-2 éthyl ] N'-méthyl nitro-2 éthène diamine-1,1 (formule 15)

On chauffe à reflux pendant 9 h 30 une solution de 3,3 g (0,0136 mole) d'[ (éthyl-5 dihydro-5,6-4H-furo [ 2,3-c ] pyrrolyl-2) méthylthio ]-2 éthylamine et 2,25 g (0,0136 mole) de bis méthylthio-2,2' nitro éthène dans 35 cm³ d'acétonitrile ; on évapore à sec puis on reprend le résidu par 35 cm³ d'une solution de méthylamine à 33 % dans l'éthanol ; on chauffe cette solution au reflux pendant 8 heures, puis on évapore et chromatographie le résidu ainsi obtenu en solution dans le méthanol sur une colonne de 40 g de gel de silice en utilisant le méthanol comme éluant ; on disperse le résidu obtenu après évaporation du méthanol dans l'acétate d'éthyle et on essore : Poids = 1,5 g, Rdt = 33 %, F = 103 - 110°C, F = 110 - 12°C (diisopropyléther - isopropanol, 5-4)

Analyse : $C_{14}H_{22}N_4O_3S$ P.M. = 326,42

| | C | H | N | S |
|---|---|---|---|---|
| % calculé | 51,51 | 6,79 | 17,17 | 9,82 |
| % trouvé | 51,29 | 6,64 | 17,36 | 9,63 |

EXEMPLE 16 : N-Cyano N'-[ [ (éthyl-5 dihydro-5,6-4H-furo [ 2,3-c ] pyrrolyl-2) méthylthio ]-2 éthyl ] N"-méthyl guanidine (formule 16)

On chauffe au reflux pendant 6 heures une solution de 2,3 g (0,0095 mole) d'[ (éthyl-5 dihydro-5,6-4H-furo [ 2,3-c ] pyrrolyl-2) méthylthio ]-2 éthylamine et 1,4 g (0,0095 mole) de cyano imido dithio carbonate de diméthyle ; puis on évapore le solvant et on disperse le résidu dans l'éther ; on reprend le solide ainsi obtenu par 30 cm³ d'acétate d'éthyle, on chauffe au reflux puis on laisse cristalliser ; on essore le solide ainsi obtenu et on le chromatographie en solution dans un mélange acétate d'éthyle-méthanol sur une colonne de 15 g de gel de silice en utilisant l'acétate d'éthyle suivi du méthanol comme éluants ; après évaporation, on obtient un solide : Poids = 1,5 g, F = 53 - 58°C

On chauffe ensuite au reflux une solution de ce solide dans 20 cm³ d'éthanol à 33 % de méthylamine puis on évapore à sec, on disperse le résidu dans l'éther, on l'essore et on le sèche : Poids = 1,3 g, Rdt =44%, F = 125 - 128°C, F = 129 - 131°C (acétate d'éthyle - isopropanol, 3-1)

R.M.N. ($CDCl_3$)

1,2 (t) ($CH_3$-$CH_2$) ; 2,6-3,1 (m) (S-$CH_2$, $CH_3$-N, $CH_3$-$CH_2$) ;
3,5 (q) 2H ($CH_2$-NH) ; 3,8 (s) 6H ($CH_2S$, $CH_2N$) ;
5,7 (t) 1H (NH) ; 6,1 (m) 1H (NH) ; 6,2 (s) 1H (H furanne)

Analyse : $C_{14}H_{21}N_5OS$ P.M. = 307,42

| | C | H | N | S |
|---|---|---|---|---|
| % calculé | 54,69 | 6,89 | 22,78 | 10,43 |
| % trouvé | 54,55 | 6,74 | 22,60 | 10,57 |

EXEMPLE 17 : N-[ [ (Ethyl-5 dihydro-5,6-4H-furo [ 2,3-c ] pyrrolyl-2) méthylthio ]-2 éthyl ] méthylthio-1 Nitro-2 éthénamine (Formule 17)

On chauffe à reflux pendant 6 heures, une solution de 2,3 g (0,0095 mole) d'[ (éthyl-5 dihydro-5,6-4H-furo [ 2,3-c ] pyrrolyl-2) méthylthio ]-2 éthylamine et 1,6 g (0,0095 mole) de bis méthylthio-2,2' nitro éthène dans 25 cm³ d'acétonitrile ; on évapore ensuite la solution et on recristallise le résidu dans 25 cm³ d'acétate d'éthyle en présence de Norit, on essore et on sèche le solide obtenu : Poids = 1,4 g, Rdt = 43 % F = 77 - 79°C, F = 80 - 82°C (chromatographie sur 10 g de gel de silice : éluants $CH_2Cl_2$ puis méthanol)

R.M.N. ($CDCl_3$)

1,1 (t) 3H ($CH_3$-$CH_2$) ; 2,4 (s) 3H ($CH_3S$) ; 2,8 (m) 4H ($SCH_2$, $CH_2N$) ;
3,3-3,9 (m) 8H ($CH_2N$, $CH_2S$) ; 6,1 (s) 1H (=CH-$NO_2$) ;

6,5 (s) 1H (H furanne) ; 10,5 (s) 1H (NH)

Analyse : $C_{14}H_{21}N_3O_3S_2$ P.M. = 343,46

| | C | H | N | S |
|---|---|---|---|---|
| % calculé | 48,95 | 6,16 | 12,23 | 18,67 |
| % trouvé | 49,20 | 5,94 | 12,04 | 18,45 |

EXEMPLE 18 : N-[ [ (Dihydro-5,6 n.pentyl-5-4H-thiéno [ 2,3-c ] pyrrolyl-2) méthylthio ]-2 éthyl ]N'-méthyl nitro-2 éthène diamine-1,1 (Formule 18)

Préparé dans les conditions de l'exemple 3 à partir de la N-[ [ (dihydro-5,6 n.pentyl-5-4H-thiéno [ 2,3-c ] pyrrolyl-2) méthylthio]-2 éthyl ] méthylthio-1 nitro-2 éthénamine et de la méthylamine : Poids = 3,6 g, Rdt = 86 %, F = 90 - 93°C, F = 93 - 95°C (diisopropyléther - isopropanol, 1-2)

Analyse : $C_{17}H_{28}N_4O_2S_2$ P.M. = 384,55

| | C | H | N | S |
|---|---|---|---|---|
| % calculé | 53,09 | 7,34 | 14,57 | 16,68 |
| % trouvé | 53,24 | 7,26 | 14,44 | 16,75 |

EXEMPLE 19 : N-[ [ (Ethyl-5 dihydro-5,6 méthyl-3-4H-thiéno [ 2,3-c ] pyrrolyl-2) méthylthio ]-2 éthyl ] N'-méthyl nitro-2 éthène diamine-1,1 (Formule 19)

Préparé dans les conditions de l'exemple 3 à partir de la N-[ [ (Ethyl-5 dihydro-5,6 méthyl-3-4H-thiéno [ 2,3-c ] pyrrolyl-2) méthylthio ]-2 éthyl] méthylthio-1 nitro-2 éthènamine et de la méthylamine.

F = 112 - 113°C (diisopropyléther - isopropanol)

Analyse : $C_{15}H_{24}N_4O_2S_2$ P.M. = 356,5

| | C | H | N | S |
|---|---|---|---|---|
| % calculé | 50,53 | 6,78 | 15,72 | 17,99 |
| % trouvé | 50,48 | 6,72 | 15,84 | 18,22 |

EXEMPLE 20 : N-[ [ (Ethyl-5 dihydro-5,6-4H-thiéno [ 2,3-c ] pyrrolyl-2) méthylthio ]-2 éthyl ]N'-méthyl oxyde-1 thiadiazole-[ 1,2,5 ] diamine-3,4 (Formule 20)

Préparé dans les conditions de l'exemple 13 en utilisant la méthylamine au lieu de l'ammoniac. F = 144 - 146°C (isopropyléther - isopropanol 1-4)

R.M.N. ($CDCl_3$)

1,16 (t) 3H ($CH_3$-CH2) ; 2,35-3,1 (m) 7H ($SCH_2$, $CH_2N$, $CH_3N$)

3,1-4 (m) 8H ($CH_2$-S, $NCH_2$) ; 6,7 (s) 1H (H thiophène)

7,6-8,4 (s élargi) 2H (NH)

Analyse : $C_{14}H_{21}N_5OS_3$ P.M. = 371,55

| | C | H | N | S |
|---|---|---|---|---|
| % calculé | 45,25 | 5,70 | 18,85 | 25,89 |
| % trouvé | 45,27 | 5,39 | 18,74 | 25,65 |

EXEMPLE 21 : N-[[(Ethyl-5 dihydro-5,6-4H-thiéno [2,3-c] pyrrolyl-2) méthylthio]-2 éthyl] N'-propargyl oxyde-1 thiadiazole [1,2,5] diamine-3,4 (Formule 21).

On agite à température ambiante pendant 6 heures un mélange de 1,7 g (0,0089 mole) de diéthoxy-3,4 thiadiazole [1,2,5] oxyde-1 et 0,5 g (0,0089 mole) de propargylamine en solution dans 20 $cm^3$ de méthanol ; on évapore à sec puis on ajoute entre 5 et 10°C, 2,15 g (0,0089 mole) d'[(éthyl-5 dihydro-5,6-4H-thiéno [2,3-c] pyrrolyl-2) méthylthio]- 2 éthylamine en solution dans 20 $cm^3$ de méthanol ; on agite cette solution à température ambiante pendant 17 heures puis on essore le précipité formé : Poids = 1,8 g Rdt = 51 % F = 164 - 166°C (déc.)
F = 165 - 166°C (méthanol - DMF, 1,8-1)

Analyse : $C_{16}H_{21}N_5OS_3$ P.M. = 395,57

| | C | H | N | S |
|---|---|---|---|---|
| % calculé | 48,58 | 5,35 | 17,71 | 24,32 |
| % trouvé | 48,58 | 5,41 | 17,63 | 24,40 |

EXEMPLE 22 : N-[[(Dihydro-5,6 n. propyl-5-4H-thiéno [2,3-c] pyrrolyl-2) méthylthio]-2 éthyl] oxyde-1 thiadiazole [1,2,5] diamine-3,4 (Formule 22).

A une solution de 2,4 g (0,0126 mole) de diéthoxy-3,4 thiadiazole [1,2,5] oxyde-1 dans 25 $cm^3$ de méthanol, on ajoute entre 5 et 10°C une solution de 3,25 g (0,0126 mole) de [(dihydro-5,6 n. propyl-5-4H-thiéno [2,3-c] pyrrolyl-2 méthylthio]-2 éthylamine dans 25 $cm^3$ de méthanol ; on agite ce mélange pendant 20 heures à la température ambiante puis on y fait barboter, pendant 10 mn entre 0 et 5°C, un courant d'ammoniac ; on poursuit l'agitation pendant 4 heures à température ambiante puis on refroidit à 0°C et on essore le précipité formé : Poids = 2,4 g Rdt = 51%
F = 178°C (déc) F = 179°C (méthanol - DMF, 1-1)

Analyse : $C_{14}H_{21}N_5OS_3$ P.M. = 371,55

| | C | H | N | S |
|---|---|---|---|---|
| % calculé | 45,25 | 5,70 | 18,85 | 25,89 |
| % trouvé | 45,41 | 5,75 | 18,70 | 25,64 |

Les composés des exemples 23 et 24 suivants sont préparés selon le mode opératoire décrit à l'exemple 2 à partir des [ (dihydro-5,6-4H-thiéno [2,3-c ] pyrrolyl-2) méthylthio ]-2 éthylamines appropriées :

EXEMPLE 23 : N-[ [ (Ethyl-5 dihydro-5,6 méthyl-3-4H-thiéno [ 2,3-c ] pyrrolyl-2) méthylthio ]-2 éthyl ] méthylthio-1 nitro-2 éthène amine (Formule 23) $C_{15}$ $H_{23}$ $N_3$ $O_2$ $S_3$, P.M. = 373,55, F = 74 - 77°C (diisopropyléther - isopropanol)

R.M.N. ($CDCl_3$) : 2,46 (s) 3H ($CH_3S$)

EXEMPLE 24 : N-[ [ (Dihydro-5, 6 n. pentyl-5-4H-thiéno [ 2,3-c ] pyrrolyl-2) méthylthio ]-2 éthyl ] méthylthio-1 nitro-2 éthène amine (Formule 24) $C_{17}$ $H_{27}$ $N_3$ $O_2$ $S_3$, P.M. = 401,61, F = 72 - 74°C (isopropanol)

R.M.N. ($CDCl_3$) : 2,46 (s) 3H ($CH_3S$)

EXEMPLE 25 : N-Cyano N'-[ [ (dihydro-5,6 phényl-5-4H-thiéno [ 2,3-c ] pyrrolyl-2) méthylthio-2 éthyl ] S-méthyl isothiourée (Formule 25)

$C_{18}$ $H_{20}$ $N_4$ $S_3$, P.M. = 388,57

On chauffe au reflux un mélange de 2,6 g (0,0094 mole) de [ (dihydro-5,6 phényl-5-4H-thiéno [ 2,3-c ] pyrrolyl-2) méthylthio ]-2 éthylamine et de 1,4 g (0,0094 mole) de cyano imido dithiocarbonate de diméthyle dans 50 $cm^3$ d'acétonitrile ; on essore à froid le solide obtenu, on le lave au diisopropyléther et on le sèche : Poids = 3g   Rdt = 82 %

F = 189 - 193°C   F = 195 - 197°C ($H_2O$ - DMF, 4-15)

I.R. (KBr) : $\nu$CN 2180 $cm^{-1}$

EXEMPLE 26 : N-Cyano N'-[ [ (éthyl-5 dihydro-5,6-4H-thiéno [ 2,3-c ] pyrrolyl-2) méthylthio ]-2 éthyl ] S-méthyl isothiourée (Formule 26)

$C_{14}$ $H_{20}$ $N_4$ $S_3$, P.M. = 340,53

Préparé selon le mode opératoire de l'exemple 25, à partir de l'[(éthyl-5 dihydro-5,6-4H-thiéno [ 2,3-c ] pyrrolyl-2) méthylthio ]-2 éthylamine :

F = 112 - 114°C

I.R. (KBr) : $\nu$CN = 2170 $cm^{-1}$

EXEMPLE 27 : N-[ [ (Ethyl-5 dihydro-5,6-4H-thiéno [ 2,3-c ] pyrrolyl-2) méthoxy ]-2 éthyl ] oxyde-1 thiadiazole [ 1,2,5 ] diamine-3,4 (formule 27)

A une solution de 3,2 g (0,0168 mole) de diéthoxy-3,4 thiadiazole [ 1, 2, 5 ] oxyde-1 dans 30 $cm^3$ de méthanol, on ajoute entre 5 et 10°C une solution de 3,8 g (0,0168 mole) d'[ (éthyl-5 dihydro-5,6-4H-thiéno [2,3-c] pyrrolyl-2) méthoxy ]-2 éthylamine dans 20 $cm^3$ de méthanol ; on

agite le mélange pendant 9 heures à température ambiante puis on y fait barboter, entre 0 et 5°C pendant 10 mn, un courant d'ammoniac ; on poursuit l'agitation à température ambiante pendant 5 heures puis on évapore à sec ; on reprend le résidu par l'eau et le chloroforme, on sèche la phase organique sur sulfate de sodium, on évapore le chloroforme, on disperse le résidu dans la quantité suffisante d'isopropanol, on essore, lave au diisopropyléther et sèche :

Poids = 1,5 g  Rdt = 26 %  F = 144 - 146°C (déc.)

F = 149 - 150°C (méthanol)

Analyse : $C_{13} H_{19} N_5 O_2 S_2$  P.M. = 341,45

| | C | H | N | S |
|---|---|---|---|---|
| % calculé | 45,73 | 5,61 | 20,51 | 18,78 |
| % trouvé | 46,01 | 5,49 | 20,25 | 18,68 |

EXEMPLE 28 : N-[ [ (n.Butyl-5 dihydro-5,6-4H-thiéno [ 2,3-c ] pyrrolyl-2) méthylthio ]-2 éthyl ] oxyde-1 thiadiazole [ 1,2,5 ] diamine-3,4 (Formule 28)

A une solution de 4,1 g (0,0214 mole) de diéthoxy-3,4 thiadiazole [1,2,5] oxyde-1 dans 40 $cm^3$ de méthanol on ajoute entre 0 et 10°C une solution de 5,8 g (0,0214 mole) de [ (n.butyl-5 dihydro-5,6-4H-thiéno [ 2,3-c ] pyrrolyl-2) méthylthio ]-2 éthylamine dans 40 $cm^3$ de méthanol ; on agite le mélange pendant 17 heures à température ambiante puis on y fait barboter, entre 0° et 5°C, un courant d'ammoniac pendant 10 mn ; on agite ensuite pendant 4 heures à la température ambiante, on essore le solide formé et on le sèche : Poids = 5,1 g  Rdt = 62 %

F = 183 - 4°C ($C_2H_5OH$-DMF, 2-1)

Analyse : $C_{15} H_{23} N_5 O S_3$  P.M. = 285,57

| | C | H | N | S |
|---|---|---|---|---|
| % calculé | 46,72 | 6,01 | 18,17 | 24,95 |
| % trouvé | 46,88 | 5,67 | 18,08 | 24,49 |

Les composés des exemples 29 à 33 suivants sont préparés selon le mode opératoire de l'exemple 28 à partir des [ (dihydro-5,6-4H-thiéno [ 2,3-c] pyrrolyl-2) méthylthio ]-2 alkylamines appropriées et du diéthoxy-3,4 thiadiazole [ 1,2,5 ] oxyde-1.

EXEMPLE 29 : N-[ [ (Isobutyl-5 dihydro-5,6-4H-thiéno [ 2,3-c ] pyrrolyl-2) méthylthio ]-2 éthyl ] oxyde-1 thiadiazole [ 1,2,5 ] diamine-3,4 (Formule 29)

Rdt = 57 %  F = 178 - 9°C (éthanol - DMF, 7-4)

Analyse : $C_{19}H_{23}N_5OS_3$ P.M. = 385,57

| | C | H | N | S |
|---|---|---|---|---|
| % calculé | 46,72 | 6,01 | 18,17 | 24,95 |
| % trouvé | 46,91 | 6,06 | 17,93 | 24,67 |

EXEMPLE 30 : N- ⌈ ⌈ (n.Hexyl-5 dihydro-5,6-4H-thiéno ⌈ 2,3-c ⌋ pyrrolyl-2) méthylthio ⌋-2 éthyl ⌋ oxyde-1 thiadiazole ⌈ 1,2,5 ⌋ diamine-3,4 (Formule 30)

F = 180 - 181°C (éthanol - DMF, 7-4) Rdt = 73 %

Analyse : $C_{17}H_{27}N_5OS_3$ P.M. = 413,63

| | C | H | N | S |
|---|---|---|---|---|
| % calculé | 49,36 | 6,58 | 16,93 | 23,26 |
| % trouvé | 49,41 | 6,59 | 16,63 | 22,95 |

EXEMPLE 31 : N-⌈ ⌈ (Dihydro-5,6 n.pentyl-5-4H-thiéno ⌈ 2,3-c ⌋ pyrrolyl-2) méthylthio ⌋-2 éthyl ⌋ oxyde-1 thiadiazole ⌈ 1,2,5 ⌋ diamine-3,4 (Formule 31) F = 172 - 173°C (éthanol), Rdt = 60 %

Analyse : $C_{16}H_{25}N_5OS_3$ P.M. = 399,60

| | C | H | N | S |
|---|---|---|---|---|
| % calculé | 48,09 | 6,31 | 17,53 | 24,07 |
| % trouvé | 48,15 | 6,33 | 17,32 | 23,80 |

EXEMPLE 32 : N-⌈ ⌈ (Ethyl-5 dihydro-5,6-4H-thiéno ⌈ 2,3-c ⌋ pyrrolyl-2) méthylthio ⌋-3 propyl ⌋ oxyde-1 thiadiazole ⌈ 1,2,5 ⌋ diamine-3,4 (Formule 32) F = 157 - 158°C (isopropanol) Rdt = 27 %

Analyse : $C_{14}H_{21}N_5OS_3$ P.M. = 371,55

| | C | H | N | S |
|---|---|---|---|---|
| % calculé | 45,25 | 5,70 | 18,85 | 25,89 |
| % trouvé | 44,94 | 5,60 | 18,98 | 25,60 |

EXEMPLE 33 : N-⌈ ⌈ ⌈ Dihydro-5,6 (méthoxy-2 éthyl)-5-4H-thiéno ⌈ 2,3-c ⌋ pyrrolyl-2 ⌋ méthylthio ⌋-2 éthyl ⌋ oxyde-1 thiadiazole ⌈ 1,2,5 ⌋ diamine-3,4 (Formule 33) F = 174 - 176°C (éthanol) Rdt = 64 %

Analyse : $C_{14}H_{21}N_5O_2S_3$ P.M. = 387,55

| | C | H | N | S |
|---|---|---|---|---|
| % calculé | 43,39 | 5,46 | 18,07 | 24,82 |
| % trouvé | 43,19 | 5,38 | 18,18 | 24,84 |

EXEMPLE 34 : Amino-3 ⌈ ⌈ (dihydro-5,6 n.propyl-5-4H-thiéno ⌈ 2,3-c ⌋ pyrrolyl-2) méthylthio ⌋-2 éthylamino ⌋-4 cyclobutène dione-1,2 (Formule 34)

A une solution de 2,6 g (0,01 mole) de N-⌈ (dihydro-5,6 n.propyl-5-4H-thiéno ⌈2,3-c⌋ pyrrolyl-2) méthylthio ⌋-2 éthylamine dans 20 $cm^3$ de méthanol, on ajoute entre 5 et 10°C, une solution de 1,4 g (0,01 mole) de diméthoxy-3,4 cyclobutène dione-1,2 dans 20 $cm^3$ de méthanol ; on agite le mélange à température ambiante pendant 48 heures puis on y fait barboter, entre 5 et 10°C, pendant 15 mn un courant d'ammoniac après quoi on poursuit l'agitation pendant 4 heures à température ambiante, on filtre le solide obtenu, on le lave à l'éther et on le sèche puis on le recristallise dans un mélange de 20 ml d'éthanol et 20 ml de D.M.F. : Poids = 0.9 g Rdt = 25 % F > 320°C

Analyse : $C_{16} H_{21} N_3 O_2 S_2$ P.M. = 351,48

| | C | H | N | S |
|---|---|---|---|---|
| % calculé | 54,67 | 6,02 | 11,96 | 18,25 |
| % trouvé | 54,59 | 5,92 | 11,94 | 18,52 |

EXEMPLE 35 : N-⌈ ⌈ (Dihydro-5,6 n.propyl-5-4H-furo ⌈ 2,3-c ⌋ pyrrolyl-2) méthylthio ⌋-2 éthyl ⌋ oxyde-1 thiadiazole ⌈1,2,5⌋ diamine-3,4 (Formule 35)

$C_{14} H_{21} N_5 O_2 S_2$ P.M.= 355,48

A une solution de 5,3 g (0,028 mole) de diéthoxy-3,4 thiadiazole ⌈1,2,5⌋ oxyde-1 dans 50$cm^3$ de méthanol on ajoute entre 5 et 10°C une solution de 6,7 g (0,028 mole) de ⌈ (dihydro-5,6 n.propyl-5-4H furo ⌈ 2,3-c ⌋ pyrrolyl-2) méthylthio ⌋-2 éthylamine dans 50 $cm^3$ de méthanol ; on agite ensuite le mélange pendant 20 heures à température ambiante puis on y fait barboter, entre 5 et 10°C, un courant d'ammoniac ; on poursuit l'agitation pendant 4 heures à température ambiante puis on évapore à sec, on disperse le résidu ainsi obtenu dans l'éther, on l'essore et on le sèche : Poids = 7 g Rdt = 70 % F = 146 - 8°C, F = 154 - 6°C (après 3 chromatographies sur colonne de gel de silice avec pour éluants un mélange chloroforme-éthanol, 95-5 puis 90-10).

Analyse

| | C | H | N | S |
|---|---|---|---|---|
| % calculé pour | | | | |
| $C_{14}H_{21}N_5O_2S_2+1/2H_2O$ | 46,16 | 6,09 | 19,22 | 17,59 |
| % trouvé | 46,46 | 5,87 | 19,38 | 17,91 |

EXEMPLE 36 : Ethyl-5 dihydro-5,6-4H-furo ⌈2,3-c⌋ pyrrole-2 carboxylate de méthyle (Formule 36)

On agite pendant 4 jours, sous atmosphère d'azote, un mélange de 44,8 g (0,167 mole) de bromométhyl-5 chlorométhyl-4 furanne-2 carboxylate de méthyle et 22,6 g (0,502 mole) d'éthylamine en solution dans 5 l d'acétonitrile ; on évapore ensuite le solvant et l'excès d'éthylamine qui n'a pas réagi, on reprend le résidu par de l'acide chlorhydrique 5N, on élimine un insoluble par filtration, on lave le filtrat à l'éther puis on le neutralise par addition de bicarbonate de sodium ; on extrait à l'éther, on lave cette phase éthérée à l'eau puis on la sèche sur sulfate de sodium ; successivement après évaporation de l'éther, on reprend le résidu à l'hexane, on chauffe à reflux en présence de Norit, on filtre, on décante une huile puis on évapore partiellement l'hexane, on filtre et évapore à sec : Poids = 13 g, Rdt = 40 %, F = 52 - 4°C (diisopropyléther)

R.M.N.

1,1 (t) 3H $CH_3$-$CH_2$ ; 2,8 (q) 2H $CH_2N$ ; 3 (s) 3H $CH_3O$

3-3,9 (m) 4H $NCH_2$ ; 7,1 (s) 1H H aromatique

Analyse : $C_{10}$ $H_{13}$ $NO_3$ P.M. = 195,21

| | C | H | N |
|---|---|---|---|
| % calculé | 61,52 | 6,71 | 7,18 |
| % trouvé | 61,41 | 6,65 | 7,23 |

EXEMPLE 37 : Ethyl-5 dihydro-5,6-4H-furo [ 2,3-c ] pyrrole-2 méthanol (Formule 37)

A une solution de 3 g (0,079 mole) d'hydrure de lithium et d'aluminium en solution dans 150 $cm^3$ d'éther, on ajoute à température ambiante, 11,7 g (0,06 mole) d'éthyl-5 dihydro-5,6-4H-furo [ 2,3-c ] pyrrole-2 carboxylate de méthyle en solution dans 70 $cm^3$ d'éther ; on chauffe au reflux pendant 2 h 30, puis on ajoute, au mélange refroidi, de l'eau et de la soude diluée ; on décante la phase éthérée, on extrait le précipité minéral à l'éther ; les différentes fractions d'éther sont réunies et séchées sur sulfate de sodium puis évaporées à sec pour fournir un solide qu'on disperse sous diisopropyléther : Poids = 5,1 g, F = 67 - 69°, Rdt = 51 %

R.M.N. ($CDCl_3$)

1,2 (t) 3H $CH_3$- ; 2,8 (q) 2H $CH_2$-$CH_3$ ; 3,8 (s) 4H $CH_2$-N ;

3,9 (m) 1H OH ; 4,6 (s) 2H $CH_2O$ ; 6,2 (s) 1H H aromatique

Analyse : $C_{19}$ $H_{13}$ $NO_2$ P.M. = 167,20

| | C | H | N |
|---|---|---|---|
| % calculé | 64,65 | 7,83 | 8,37 |
| % trouvé | 64,43 | 7,89 | 8,42 |

EXEMPLE 38 : [ (Ethyl-5 dihydro-5,6-4H-furo [ 2,3-c ] pyrrolyl-2) méthylthio ]-2 éthylamine (Formule 38) $C_{11}$ $H_8$ $N_2$ OS, P.M. = 242,34

A une solution de 2,5 g (0,022 mole) de chlorhydrate de cystéamine dans 8,8 $cm^3$ d'acide chlorydrique concentré, on ajoute par petites portions tout en refroidissant entre 0 et 4°C 3,7 g (0,022 mole) d'éthyl-5 dihydro-5,6-4H-furo [ 2,3-c ] pyrrole-2 méthanol ; on agite ensuite le mélange pendant 2 heures entre 4 et 5°C, puis on le conserve pendant 48 heures à -5°C après quoi on l'alcalinise par addition de bicarbonate de sodium et on extrait à l'éther ; après séchage de la phase éthérée sur sulfate de sodium puis évaporation, on obtient une huile qu'on utilise telle quelle dans l'étape suivante : Poids = 3,3 g, Rdt = 62 %

EXEMPLE 39 : Dihydro-5,6 n.propyl-5 furo [ 2,3-c ] pyrrole-2 carboxylate de méthyle (Formule 39) $C_{11}$ $H_{15}$ $NO_3$, P.M. = 209,24

On agite pendant 7 jours à température ambiante, un mélange de 69 g (0,258 mole) de bromométhyl-5 chlorométhyl-4 furanne-2 carboxylate de méthyle et 46 g (0,775 mole) de n. propylamine dans 7,67 litres d'acétonitrile ; on filtre le mélange puis on évapore à sec le filtrat, on reprend le résidu obtenu par de l'acide chlorhydrique 5N, on élimine l'insoluble par essorage, on alcalinise le filtrat par addition de bicarbonate de sodium ; on extrait à l'éther, sèche sur sulfate de sodium et évapore à sec ; on obtient ainsi une huile qu'on utilise telle quelle dans la suite de la synthèse : Poids = 31,4 g, Rdt = 58 %

EXEMPLE 40 : Dihydro-5,6 n. propyl-5 furo [ 2,3-c ] pyrrole-2 méthanol (Formule 40) $C_{10}$ $H_{15}$ $NO_2$ P.M. = 181,23

A une solution de 7 g (0,1845 mole) d'hydrure de lithium et d'aluminium dans 350 $cm^3$ d'éther, on ajoute au reflux, 31,4 g (0,15 mole) de dihydro-5,6 n.propyl-5 furo [ 2,3-c ] pyrrole-2 carboxylate de méthyle en solution dans 176 $cm^3$ d'éther ; après quoi, on chauffe au reflux le mélange pendant 4 heures puis on le traite comme dans l'exemple 37 ; on isole ainsi un solide : Poids = 11 g, Rdt = 40 %, F = 60 -62°C

R.M.N. ($CDCl_3$)

0,9 (t) 3H ($CH_3$) ; 1,5 (sext.) 2H ($\underline{CH_2}CH_3$) ; 2,7 (t) 2H ($N\underline{CH_2}CH_2-$) ; 3,7 (s) 4H ($CH_2N$) ; 3,9 (s) 1H (OH) ; 4,5 (s) 2H ($\underline{CH_2}OH$) ; 6,1 (s) 1H arom.

EXEMPLE 41 : [ (Dihydro-5,6 n.propyl-5-4H-furo [ 2,3-c ] pyrrolyl-2) méthylthio ]-2 éthylamine (Formule 41), $C_{12}$ $H_{20}$ $N_2OS$ P.M. = 240,37

Préparée dans les conditions de l'exemple 38 à partir de chlorhydrate de cystéamine et de dihydro-5,6 n.propyl-5 furo [ 2,3-c ] pyrrole -2 méthanol ; on traite le mélange réactionnel de la façon suivante : alcalinisation à la soude 10 N, extraction à l'éther, nouvelle extraction de la phase aqueuse par un mélange éther-méthanol après saturation par de la soude en pastilles ; les deux phases organiques sont réunies, séchées sur sulfate de sodium, évaporées et distillées : $Eb_2$ = 148 - 150°C, Poids = 6,7 g, Rdt = 46 %

R.M.N. ($CDCl_3$)

0,9 (t) 3H ($CH_3$) ; 1,2-1,8 (m) 2H ($CH_3\underline{CH_2}$) ; 1,5 (s) 1H ($NH_2$) ;
2,5-3 (m) 6H ($CH_2$) ; 3,7 (q + s) 6H ($CH_2S$ et $CH_2N$) ; 6,1 (s) 1H (arom.)

EXEMPLE 42 : Ethyl-5 dihydro-5,6-4H-thiéno [ 2,3-c ] pyrrole-2 carboxylate d'éthyle (Formule 42), $C_{11}H_{15}NO_2S$, P.M. = 225,3

Dans un réacteur placé sous atmosphère d'azote, on dissout 126,6g (0,5 mole) de bis chlorométhyl-4,5 thiophène-2 carboxylate d'éthyle dans 15 litres d'acétonitrile puis on y ajoute 67,6 g (1,5 mole) d'éthylamine ; on agite le mélange pendant 8 jours à température ambiante puis on filtre, on évapore à sec le filtrat, on dissout le résidu obtenu dans la quantité suffisante d'acide chlorhydrique 1,5 N, on filtre la solution, on la lave à l'éther puis on alcalinise par addition de carbonate de potassium ; on extrait à l'éther ; après séchage de la phase éthérée sur sulfate de sodium puis évaporation, on obtient une huile qu'on distille sous pression réduite : $Eb_{1,5}$ = 122 - 125°C, Poids = 65,4 g, Rdt = 58 %,
C.P.V. SE 30 - 1 mètre - T = 230°C : 1 seul pic

Selon le mode opératoire de l'exemple 42, à partir de l'amine appropriée et du bis chlorométhyl-4,5 thiophène-2 carboxylate d'éthyle ou de méthyle, on prépare les esters suivants :

EXEMPLE 43 : Dihydro-5,6 méthyl-5-4H-thiéno [ 2,3-c ] pyrrole-2 carboxylate d'éthyle (Formule 43) $C_{10}H_{13}NO_2S$ P.M. = 211,27
utilisé brut dans l'étape suivante.

EXEMPLE 44 :Benzyl-5 dihydro-5,6-4H-thiéno [ 2,3-c ] pyrrole-2 carboxylate d'éthyle (Formule 44) $C_{16}H_{17}NO_2S$ P.M. = 287,28
F = 223 - 4°C

EXEMPLE 45 : Dihydro-5,6 isopropyl-5-4H-thiéno [ 2,3-c ] pyrrole-2 carboxylate d'éthyle (Formule 45) $C_{12}H_{17}NO_2S$ P.M. = 239,33
Utilisé brut dans l'étape suivante.

EXEMPLE 46 : Dihydro-5,6 phényl-5-4H-thiéno [ 2,3-c ] pyrrole-2 carboxylate d'éthyle (Formule 46) $C_{15}H_{15}NO_2S$ P.M. = 273,34

F = 147 - 150°C

EXEMPLE 47 : Dihydro-5,6 n.pentyl-5-4H-thiéno [ 2,3-c ] pyrrole-2 carboxylate d'éthyle (Formule 47) $C_{14}H_{21}N_2O_2S$ P.M. = 267,38

$Eb_1$ = 136 - 145°C

EXEMPLE 48 : Dihydro-5,6 n.propyl-5-4H-thiéno [ 2,3-c ] pyrrole-2 carboxylate d'éthyle (Formule 48) $C_{12}H_{17}NO_2S$ P.M. = 239,44

Utilisé brut dans l'étape suivante.

EXEMPLE 49 : n.Butyl-5 dihydro-5,6-4H-thiéno [ 2,3-c ] pyrrole-2 carboxylate de méthyle (Formule 49) $C_{12}H_{17}NO_2S$ P.M. = 239,32

$Eb_2$ = 138 - 145°C

EXEMPLE 50 : Isobutyl-5 dihydro-5,6-4H-thiéno [ 2,3-c ] pyrrole-2 carboxylate de méthyle (Formule 50) $C_{12}H_{17}NO_2S$ P.M. = 239,23

$Eb_{1,5}$ = 120 - 125°C

EXEMPLE 51 : n.Hexyl-5 dihydro-5,6-4H-thiéno [ 2,3-c ] pyrrole-2 carboxylate de méthyle (Formule 51) $C_{14}H_{21}NO_2S$, P.M. = 267,33

Solide amorphe

EXEMPLE 52 : Dihydro-5,6 (méthoxy-2 éthyl)-5-4H-thiéno [ 2,3-c ] pyrrole-2 carboxylate de méthyle (Formule 52) $C_{11}H_{15}NO_3S$

P.M. = 241,30. Utilisé brut

EXEMPLE 53 : Ethyl-5 dihydro-5,6-4H-thiéno [ 2,3-c ] pyrrole-2 méthanol (Formule 53)

A une solution de 20 g (0,527 mole) d'hydrure de lithium et d'aluminium dans 1 litre d'éther placée sous atmosphère d'azote, et chauffée au reflux, on ajoute une solution de 65,1 g (0,289 mole) d'éthyl-5 dihydro-5,6-4H-thiéno [ 2,3-c ] pyrrole-2 carboxylate d'éthyle dans 400 $cm^3$ d'éther ; on poursuit le chauffage à reflux pendant 4 heures, puis on refroidit le mélange et on y introduit goutte à goutte, 120 $cm^3$ d'eau puis 100 $cm^3$ d'une lessive de soude à 30 % ; on décante la phase organique, on y joint l'éther d'extraction du précipité formé, on sèche le tout sur sulfate de sodium, on évapore à sec et on disperse le résidu solide ainsi obtenu sous hexane, on l'essore et on le sèche :

Poids = 50 g, Rdt = 88 %, F = 85 - 87°C (hexane - isopropanol, 15-1)

R.M.N. ($CDCl_3$)

1,13 (t) 3H ($CH_3$) ; 2,8 (q) 2H ($CH_2$-$CH_3$) ; 3,85 (m) 4H ($CH_2$-N) ; 4,65 (s) 2H ($CH_2O$) ; 5 (s) 1H (OH) ; 6,55 (s) 1H (H thiophène)

Analyse : $C_9 H_{13} NOS$ P.M. = 183,16

| | C | H | N | S |
|---|---|---|---|---|
| % calculé | 58,98 | 7,15 | 7,64 | 17,50 |
| % trouvé | 58,92 | 7,19 | 7,61 | 17,35 |

Selon le mode opératoire de l'exemple 53 à partir de l'ester approprié, on prépare les alcools suivants :

EXEMPLE 54 : Dihydro-5,6 méthyl-5-4H-thiéno [ 2,3-c ] pyrrole-2 méthanol (Formule 54) $C_8 H_{11} NOS$ P.M. = 169,24

F = 105 - 107°C diisopropyléther - isopropanol

EXEMPLE 55 : Dihydro-5,6 isopropyl-5-4H-thiéno [ 2,3-c ] pyrrole-2 méthanol (Formule 55) $C_{10} H_{15} NOS$ P.M. = 197,29

F = 118- 120°C (chromatographie sur alumine, éluant $CHCl_3$)

EXEMPLE 56 : Benzyl-5 dihydro-5,6-4H-thiéno [ 2,3-c ] pyrrole-2 méthanol (Formule 56) $C_{14} H_{15} NOS$ P.M. = 245,33

F = 132 - 133°C

EXEMPLE 57 : Dihydro-5,6 phényl-5-4H-thiéno [ 2,3-c ] pyrrole-2 méthanol (Formule 57) F = 197 - 198°C (THF)

Analyse : $C_{13} H_{13} NOS$ P.M. = 231,30

| | C | H | N | S |
|---|---|---|---|---|
| % calculé | 67,57 | 5,66 | 6,06 | 13,86 |
| % trouvé | 67,44 | 5,65 | 6,13 | 13,77 |

EXEMPLE 58 : Dihydro-5,6 n.propyl-5-4H-thiéno [ 2,3-c ] pyrrole-2 méthanol (Formule 58) $C_{10} H_{15} NOS$ P.M. = 197,29

Utilisé brut.

EXEMPLE 59 : Dihydro-5,6 n.pentyl-5-4H-thiéno [ 2,3-c ] pyrrole-2 méthanol (Formule 59) $C_{12} H_{19} NOS$ P.M. = 295,34

Utilisé brut

EXEMPLE 60 : Ethyl-5 dihydro-5,6 méthyl-3-4H-thiéno [ 2,3-c ] pyrrole-2 méthanol (Formule 60) $C_{10} H_{15} NOS$ P.M. = 197,29

Utilisé brut.

EXEMPLE 61 : Butyl-5 dihydro-5,6-4H-thiéno [ 2,3-c ] pyrrole-2 méthanol (Formule 61) $C_{11} H_{17} NOS$ P.M. = 211,32

F = 78 - 80°C

EXEMPLE 62 : Isobutyl-5 dihydro-5,6-4H-thiéno [ 2,3-c ] pyrrole-2 méthanol (Formule 62) $C_{11} H_{17} NOS$ P.M. = 211,32

F = 73 - 75°C

EXEMPLE 63 : n.Hexyl-5 dihydro-5,6-4H-thiéno [2,3-c] pyrrole-2 méthanol (Formule 63) $C_{13}H_{21}NOS$ P.M. = 239,37
F = 70 - 72°C

EXEMPLE 64 : Dihydro-5,6 (méthoxy-2 méthyl)-5-4H-thiéno [2,3-c] pyrrole-2 méthanol (Formule 64) $C_{10}H_{15}NO_2S$ P.M. = 213,29
F = 76 - 78°C

EXEMPLE 65 : Chlorométhyl-2 éthyl-5 dihydro-5,6-4H-thiéno [2,3-c] pyrrole, chlorhydrate (Formule 65) $C_9H_{13}Cl_2NS$ P.M. = 238,17

A une solution de 45 g (0,245 mole) d'éthyl-5 dihydro-5,6-4H-thiéno [2,3-c] pyrrole-2 méthanol dans 500 $cm^3$ de chloroforme, on ajoute à température ambiante (utilisation d'un mélange réfrigérant), 35 g (0,29 mole) de chlorure de thionyle ; on agite ensuite la solution pendant 2 heures à température ambiante puis on évapore le solvant et l'excès de chlorure de thionyle n'ayant pas réagi ; on disperse le résidu ainsi obtenu dans 200 $cm^3$ d'isopropanol, on essore et on sèche : Poids = 43,3 g
Rdt = 74 % F = 151 - 153°C (déc.)

Dans les conditions de l'exemple 65, on prépare à partir des alcools appropriés, les halogénures correspondants des exemples 66 à 68 suivants:

EXEMPLE 66 : Chlorométhyl-2 dihydro-5,6 méthyl-5-4H-thiéno [2,3-c] pyrrole, chlorhydrate (Formule 66) $C_8H_{11}Cl_2NS$ P.M. = 224,15
F = 173 - 176°C (dec) (isopropanol-méthanol)

EXEMPLE 67 : Benzyl-5 chlorométhyl-2 dihydro-5,6-4H-thiéno [2,3-c] pyrrole, chlorhydrate (Formule 67) $C_{14}H_{15}Cl_2NS$ P.M. = 300,24
F = 146 - 148°C

EXEMPLE 68 : Chlorométhyl-2 dihydro-5,6 isopropyl-5-4H-thiéno [2,3-c] pyrrole, chlorhydrate (Formule 68) $C_{10}H_{15}Cl_2NS$ P.M. = 252,12
F = 148 - 150°C

EXEMPLE 69 : [(Ethyl-5 dihydro-5,6-4H-thiéno [2,3-c] pyrrolyl-2) méthylthio]-2 éthylamine (Formule 69) $C_{11}H_{18}N_2S_2$ P.M. = 242,40

A une solution de 22,8 g (0,2 mole) de chlorhydrate de cystéamine dans 400 $cm^3$ d'acide chlorhydrique concentré, on ajoute lentement entre 0 et 5°C, 36,1 g (0,197 mole) d'éthyl-5 dihydro-5,6-4H-thiéno [2,3-c] pyrrole-2 méthanol ; on agite ensuite le mélange pendant 3 h 30 à température ambiante puis on neutralise à la soude, on extrait à l'éther, on sèche la phase éthérée sur sulfate de sodium et on évapore, on obtient ainsi une huile qu'on distille sous pression réduite :
$Eb_{0,7}$ = 149 - 155°C Poids = 30,9 g Rdt = 65 %

R.M.N. ($CDCl_3$)
1-1,4 (m) 5H ($CH_3$, $NH_2$) ; 2,2-3,1 (m) 6H (S-$CH_2$, $CH_2N$) ;
3,86 (s élargi) 6H ($NCH_2$, $CH_2S$) ; 6,7 (s) 1H (H thiophène)

EXEMPLE 70 : [ (Dihydro-5,6 méthyl-5-4H-thiéno [ 2,3-c ] pyrrolyl-2) méthylthio ]-2 éthylamine (Formule 70) $C_{10} H_{16} N_2S_2$ P.M. = 228,38

A une solution méthanolique de méthylate de sodium préparée par dissolution de 2,6 g de sodium dans 50 $cm^3$ de méthanol, on ajoute une solution de 5,9 g (0,052 mole) de chlorhydrate de cystéamine dans 50 $cm^3$ de méthanol ; on agite le mélange pendant 2 heures entre 0 et 5°C puis on y ajoute une solution de 5,8 g (0,0258 mole) de chlorhydrate de chlorométhyl-2 dihydro-5,6 méthyl-5-4H-thiéno [ 2,3-c ] pyrrole dans 60 $cm^3$ de méthanol ; on poursuit l'agitation pendant 24 heures à température ambiante, puis on filtre le mélange, on évapore le filtrat, on reprend le résidu par le chloroforme ; la phase chloroformique est ensuite lavée à l'eau, séchée sur sulfate de sodium ; puis on distille sous pression réduite l'huile obtenue après évaporation du chloroforme :

$Eb_{0,7}$ = 127 - 145°C Poids = 2,4 g Rdt = 41 %

EXEMPLE 71 : [ (Benzyl-5 dihydro-5,6-4H-thiéno [ 2,3-c ] pyrrolyl-2) méthylthio ]-2 éthylamine (Formule 71) $C_{16} H_{20} N_2 S_2$ P.M. = 304,47

Préparé selon l'exemple 70 à partir du chlorhydrate de benzyl-5 chlorométhyl-2 dihydro-5,6-4H-thiéno [ 2,3-c ] pyrrole.

$Eb_{0,7}$ = 155 - 210°C

Selon le mode opératoire de l'exemple 69, à partir des alcools appropriés on prépare les amines des exemples 72 à 79 suivants :

EXEMPLE 72 : [ (Dihydro-5,6 phényl-4H-thiéno [ 2,3-c ] pyrrolyl-2) méthylthio ]-2 éthylamine (Formule 72) $C_{15} H_{18} NS_2$ P.M. = 276,43

F = 128 - 133°C ($H_2O$ - DMF, 1-3)

EXEMPLE 73 : [ (Dihydro-5,6 n.pentyl-5-4H-thiéno [ 2,3-c ] pyrrolyl-2) méthylthio ]-2 éthylamine (Formule 73) $C_{14} H_{24} N_2S_2$, P.M. = 284,48

$Eb_2$ = 188 - 199°C

EXEMPLE 74 : [ (Ethyl-5 dihydro-5,6 méthyl-3-4H-thiéno [ 2,3-c ] pyrrolyl-2) méthylthio ]-2 éthylamine (Formule 74) $C_{12} H_{20} N_2 S_2$

P.M. = 256,43, $Eb_{1-1,5}$ = 150 - 156°C

EXEMPLE 75 : [ (Dihydro-5,6 propyl-5-4H-thiéno [ 2,3-c ] pyrrolyl-2) méthylthio ]-2 éthylamine (Formule 75) $C_{12} H_{20} N_2 S_2$, P.M. = 256,43

$Eb_{0,5-1}$ = 158 - 161°C

<u>EXEMPLE 76</u> : ⌈ (Butyl-5 dihydro-5,6-4H-thiéno ⌈ 2,3-c ⌋ pyrrolyl-2) méthylthio ⌋-2 éthylamine (Formule 76) $C_{13}H_{22}N_2S_2$ P.M. = 270,46
$Eb_{0,6-0,8}$ = 147 - 152°C
<u>EXEMPLE 77</u> : ⌈ (Isobutyl-5 dihydro-5,6-4H-thiéno ⌈ 2,3-c ⌋ pyrrolyl-2) méthylthio ⌋-2 éthylamine (Formule 77) $C_{13}H_{22}N_2S_2$ P.M. = 270,46
$Eb_{0,7-0,9}$ = 150 - 154°C
<u>EXEMPLE 78</u> : ⌈ n.Hexyl-5 dihydro-5,6-4H-thiéno ⌈ 2,3-c ⌋ pyrrolyl-2) méthylthio ⌋-2 éthylamine (Formule 78) $C_{15}H_{26}N_2S_2$ P.M. = 298,51
$Eb_{1-1,5}$ = 185 - 190°C
<u>EXEMPLE 79</u> : ⌈ Dihydro-5,6 (méthoxy-2 éthyl)-5-4H-thiéno ⌈ 2,3-c ⌋ pyrrolyl-2) méthylthio ⌋-2 éthylamine (Formule 79)
$C_{12}H_{20}N_2OS_2$ P.M. = 272,43
$Eb_{0,9-1}$ = 170 - 172°C
<u>EXEMPLE 80</u> :⌈ (Ethyl-5 dihydro-5,6-4H-thiéno ⌈ 2,3-c ⌋ pyrrolyl-2) méthoxy ⌋-2 éthylamine (Formule 80) $C_{11}H_{18}N_2OS$ P.M. = 226,34

On chauffe à 100°C pendant 6 heures un mélange de 12,5 g (0,0525 mole) de chlorhydrate de chlorométhyl-2 éthyl-5 dihydro-5,6-4H-thiéno ⌈ 2,3-c⌋ pyrrole et de 150 $cm^3$ d'amino-2 éthanol ; on évapore ensuite ce mélange à sec, on reprend le résidu à l'eau, on alcalinise cette solution aqueuse par addition de carbonate de potassium, on extrait à l'éther ; on sèche la phase éthérée sur sulfate de sodium, on évapore à sec, on isole une huile qu'on distille sous pression réduite :
$Eb_1$ = 155 - 162°C Poids = 4 g, Rdt = 33,6 %
<u>R.M.N.</u> ($CDCl_3$)
1,15 (t) 3H ($CH_3$-$CH_2$) ; 2,3 (s) 2H ($NH_2$) ; 2,6-3,1 (m) 4H ($CH_2N$) ;
3,5-4,1 (m) 8H (N-$CH_2$, $CH_2O$) ; 6,66 (s) 1H (H thiophène)

<u>EXEMPLE 81</u> : ⌈ (Ethyl-5 dihydro-5,6-4H-thiéno ⌈ 2,3-c ⌋ pyrrolyl-2) méthylthio ⌋-3 propylamine (Formule 81) $C_{12}H_{20}N_2S_2$ P.M. = 256,43

A une solution de méthylate de sodium préparée par dissolution de 3 g de sodium dans 50 $cm^3$ de méthanol, on ajoute entre 0 et 10°C, une solution de 12,4 g d'(éthyl-5 dihydro-5,6-4H-thiéno ⌈ 2,3-c ⌋ pyrrolyl-2) méthanethiol dans 30 $cm^3$ de méthanol ; on agite le mélange pendant 2 heures à la température ambiante, puis on y ajoute entre 0 et 5°C une solution de 13,6 g de chlorhydrate de bromo-3 propylamine dans 50 $cm^3$ de méthanol ; on poursuit l'agitation du mélange à la température ambiante pendant 18 heures, puis on évapore à sec, on reprend le résidu à l'éther,

on lave à l'eau la phase éthérée, on la sèche sur sulfate de sodium ; on évapore l'éther, on isole ainsi une huile qu'on distille sous pression réduite ; $Eb_{0,5}$ millibar = 154 - 157°C Poids = 7,6 g Rdt = 48 %

R.M.N. ($CDCl_3$)

1-1,4 (m) 5H ($CH_3$, $NH_2$) ; 1,45-2 (m) 2H ($CH_2$-$\underline{CH_2}$-$CH_2$-N)

2,4-3,1 (m) 6H (S-$CH_2$, $CH_2$N) ; 3,7-4,2 (m) 6H (N-$CH_2$, $CH_2$S)

6,7 (s) 1H (H thiophène)

EXEMPLE 82 : (Ethyl-5 dihydro-5,6-4H-thiéno [2,3-c] pyrrolyl-2) méthanethiol (Formule 82) $C_9H_{13}NS_2$ P.M. = 199,33

On chauffe au reflux pendant 4 heures, un mélange de 55,4 g (0,23 mole) de chlorhydrate de chlorométhyl-2 éthyl-5 dihydro-5,6-4H-thiéno [2,3-c] pyrrole et 17,7 g (0,23 mole) de thiourée dans 650 $cm^3$ d'éthanol ; on laisse refroidir le mélange, on essore le précipité formé, on le lave au diisopropyléther et on le sèche (poids = 62,3 g) ; puis on le dissout dans une solution de 28,5 g de soude en pastilles dans 250 $cm^3$ d'eau ; on chauffe cette solution au bain-marie bouillant pendant 4 heures, on filtre à froid, on ajoute au filtrat de l'acide acétique jusqu'à pH = 6 et on extrait au chloroforme ; la phase chloroformique est séchée sur sulfate de sodium, puis distillée : $Eb_{0,6-1}$ = 99 - 115°C

Poids = 25 g Rdt = 63 %

Chlorhydrate $C_9H_{14}ClNS_2$ : F = 147 - 149°C (diisopropyléther-isopropanol, 1-4)

R.M.N. ($CDCl_3$)

1,4 (t) 3H ($CH_3$-) ; 2 (t) 1H (SH) ; 3-5 (m) 8H ($CH_2$N, $CH_2$S)

6,6 (s) 1H (H thiophène) ; 13 (m) 1H ($H^+$)

Analyse :

| | C | H | N | Cl | S |
|---|---|---|---|---|---|
| % calculé | 45,84 | 5,98 | 5,94 | 15,04 | 27,2 |
| % trouvé | 45,71 | 6,02 | 5,92 | 14,96 | 26,9 |

EXEMPLE 83 : [(Ethyl-5 dihydro-5,6-4H-thiéno [2,3-c] pyrrolyl-2) méthylthio]-3 propane nitrile (Formule 83) $C_{12}H_{16}N_2S_2$

P.M. = 252,40

A une solution méthanolique de méthylate de sodium préparée par dissolution de 2,8 g de sodium dans 50 $cm^3$ de méthanol, on ajoute entre 0 et 10°C, une solution de 22,9 g d'(éthyl-5 dihydro-5,6-4H-thiéno [2,3-c] pyrrolyl-2) méthanethiol dans 100 $cm^3$ de méthanol ; on agite le mélange

pendant 2 heures entre 0 et 10°C puis on y ajoute 10,8 g de chloro-3 propane nitrile en solution dans 100 $cm^3$ de méthanol ; on poursuit l'agitation pendant 42 heures, puis on filtre le mélange, évapore le filtrat à sec, on reprend le résidu à l'éther, on lave la phase éthérée à l'eau, puis on la sèche sur sulfate de sodium ; par évaporation de l'éther on isole une huile qu'on distille : $Eb_1$ = 175 - 176°C

Poids = 19,1 g        Rdt = 66 %

<u>R.M.N.</u> ($CDCl_3$)

1,1 (t) 3H ($CH_3$) ; 2,15-3 (m) 6H ($CH_2N$, $SCH_2$, $CH_2CN$) ;
3,6-4,1 (m) 6H ($NCH_2$, $CH_2S$) ; 6,6 (s) 1H (H thiophène)

<u>EXEMPLE 84</u> : [ (Ethyl-5 dihydro-5,6-4H-thiéno [ 2,3-c ] pyrrolyl-2) méthylthio ]-3 propane imidate de méthyle (Formule 84)

$C_{13}H_{20}N_2OS_2$    P.M. = 284,44

On fait barboter pendant 4 heures, entre -5°C et +5°C, simultanément un courant de gaz chlorhydrique et d'azote dans une solution de 10 g (0,0396 mole) d'[ (éthyl-5 dihydro-5,6-4H-thiéno [ 2,3-c ]pyrrolyl-2) méthylthio] -3 propane nitrile dans 40 $cm^3$ de méthanol et 80 $cm^3$ de chloroforme ; on évapore ensuite les solvants et on verse le résidu sur 32 g de carbonate de potassium en solution dans 2 litres d'eau glacée ; on extrait immédiatement à l'éther, on sèche la phase éthérée sur sulfate de sodium et on évapore à sec ; on obtient ainsi une huile utilisée telle quelle dans la suite de la synthèse : Poids = 11,3 g        Rdt quantitatif

I.R.   $\nu$ C = N : 1650 $cm^{-1}$

<u>EXEMPLE 85</u> : Dichlorhydrate de N-[ [ (éthyl-5 dihydro-5,6-4H-thiéno [2,3-c] pyrrolyl-2) méthylthio ]-2 éthyl ] oxyde-1 thiadiazole [ 1,2,5 ] diamine-3,4 (Formule 85)

Dans une suspension de 2 g (0,0056 mole) de N-[ [ (éthyl-5, dihydro-5,6-4H-thiéno [ 2,3-c ] pyrrolyl-2) méthylthio ]-2 éthyl ] oxyde-1 thiadiazole [ 1,2,5 ] diamine-3,4 dans 25 $cm^3$ de méthanol refroidi entre 0 et 5°C on fait barboter un courant de HCl jusqu'à dissolution. On agite ensuite pendant deux heures entre 0 et 10°C, il y a apparition d'un précipité qu'on filtre, lave au diisopropyléther et sèche: Poids = 2 g, Rdt = 83 %, F = 168 - 170°C

Analyse : $C_{13} H_{21} Cl_2 N_5 OS_3$ P.M. = 430,45

| | C | H | Cl | N | S |
|---|---|---|---|---|---|
| % calculé | 36,27 | 4,92 | 16,27 | 16,47 | 22,34 |
| % trouvé | 36,33 | 4,87 | 16,23 | 16,40 | 22,14 |

EXEMPLE 86 : Dichlorhydrate de N-[ [ (dihydro-5,6 n.propyl-5-4H-thiéno [2,3-c ] pyrrolyl-2) méthylthio ]-2 éthyl ] oxyde-1 thiadiazole [ 1,2,5 ] diamine-3,4 (Formule 86)

Préparé dans les conditions de l'exemple 85 à partir de la N-[ [ (dihydro-5,6 n.propyl-5-4H-thiéno [ 2,3-c ] pyrrolyl-2) méthylthio ]-2 éthyl ] oxyde-1 thiadiazole [ 1,2,5 ] diamine-3,4

Analyse : $C_{14} H_{23} Cl_2 N_5 OS_3$ P.M. = 444,47

| | C | H | Cl | N | S |
|---|---|---|---|---|---|
| % calculé | 37,83 | 5,22 | 15,95 | 15,76 | 21,64 |
| % trouvé | 37,66 | 5,21 | 15,64 | 16,02 | 21,48 |

EXEMPLE 87 : Chlorhydrate de l'amino-3 [ [ (dihydro-5,6 n.propyl-5-4H-thiéno [ 2,3-c ] pyrrolyl-2) méthylthio ]-2 éthylamino ]-4 cyclobutène dione-1,2 (Formule 87), $C_{16} H_{22} Cl N_3 O_2 S_2$ P.M. = 387,94

Préparé dans les conditions de l'exemple 85 à partir de l'amino-3 [ [ (dihydro-5,6 n.propyl-5-4H-thiéno [ 2,3-c ] pyrrolyl-2) méthylthio ]-2 éthylamino ]-4 cyclobutène dione-1,2. F = 145 - 7°C (éthanol)

Analyse :

| | C | H | Cl | N | S |
|---|---|---|---|---|---|
| % calculé pour | | | | | |
| $C_{16}H_{22}ClN_3O_2S_2+1H_2O$ | 47,34 | 5,96 | 8,73 | 10,35 | 15,80 |
| % trouvé | 47,73 | 5,55 | 8,75 | 10,36 | 15,81 |

REVENDICATIONS

1. Thiéno et furo- [ 2,3-c ] pyrroles représentés par la formule

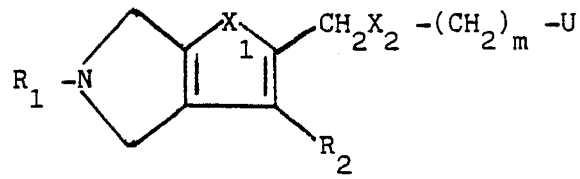

dans laquelle $X_1$ et $X_2$ désignent un atome d'oxygène ou de soufre ; $R_1$ désigne un radical alkyle linéaire ou ramifié contenant de 1 à 8 atomes de carbone, un radical alcynyle, un radical alkoxyalkyle contenant 3 à 8 atomes de carbone ou un reste aromatique ; $R_2$ désigne un atome d'hydrogène ou un radical alkyle linéaire ou ramifié ; m = 2 ou 3, quand m = 2, $X_1$ et $X_2$ désignent un atome de soufre, U représente un reste diamino-3,4- cyclobutène dione-1,2 ou aminosulfonylformamidine ; U peut aussi représenter les structures azotées

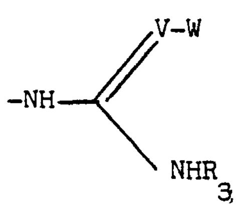 ; 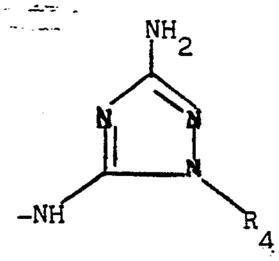 ; 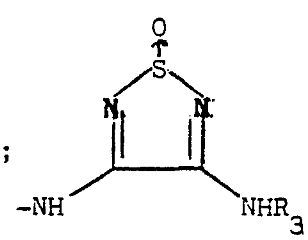

dans lesquelles V désigne un atome d'azote ou un fragment CH ; W représente $NO_2$ ou CN ; $R_3$ désigne un atome d'hydrogène, un radical alkyle linéaire ou ramifié de 1 à 8 atomes de carbone, un radical alcynyle ; $R_4$ désigne un groupement alkyle contenant de 1 à 3 atomes de carbone.

2 - Thiéno [ 2,3-c ] pyrroles selon la revendication 1,

. N-Cyano N' - [ [ (éthyl-5 dihydro-5,6-4H-thiéno [ 2,3-c ] pyrrolyl-2) méthylthio ] -2 éthyl ] N" -méthylguanidine ;

. N - [ [ (Ethyl-5 dihydro-5,6-4H-thiéno [ 2,3-c ] pyrrolyl-2) méthylthio ] -2 éthyl ] N' -méthyl nitro-2 éthène diamine-1,1 ;

. N-Cyano N' - [ [ (dihydro-5,6 isopropyl-5-4H-thiéno- [ 2,3-c ] pyrrolyl-2) méthylthio ] -2 éthyl N" -méthyl guanidine ;

. [ (Ethyl-5 dihydro-5,6-4H-thiéno [ 2,3-c ] pyrrolyl-2) méthylthio ] -3 N-sulfamoyl propanamidine ;

. N- [ [ (Ethyl-5 dihydro-5,6-4H-thiéno [ 2,3-c ] pyrrolyl-2) méthylthio] -2 éthyl ] -5 méthyl-1-1H-triazole [ 1,2,4 ] diamine-3,5 ;

. N- [ [ (Ethyl-5 dihydro-5,6-4H-thiéno [ 2,3-c ] pyrrolyl-2) méthylthio] -2 éthyl ] oxyde-1 thiadiazole [ 1,2,5 ] diamine-3,4 ;

. N- [ [ (Dihydro-5,6 n.pentyl-5-4H-thiéno [ 2,3-c ] pyrrolyl-2) méthylthio ] -2 éthyl ] N' -méthyl nitro-2 éthène diamine-1,1 ;

. N- [ [ (Ethyl-5 dihydro-5,6 méthyl-3-4H-thiéno [ 2,3-c ] pyrrolyl-2) méthylthio ] -2 éthyl ] N' -méthyl nitro-2 éthène diamine-1,1 ;

. N- [ [ (Ethyl-5 dihydro-5,6-4H-thiéno [ 2,3-c ] pyrrolyl-2) méthylthio] -2 éthyl ] N' -méthyl oxyde-1 thiadiazole [ 1,2,5 ] diamine-3,4 ;

. N- [ [ (Ethyl-5 dihydro-5,6-4H-thiéno [ 2,3-c ] pyrrolyl-2) méthylthio ] -2 éthyl ] N' -propargyl oxyde-1 thiadiazole [ 1,2,5 ] diamine-3,4 ;

. N- [ [ (Dihydro-5,6 n.propyl-5-4H-thiéno [ 2,3-c ] pyrrolyl-2) méthylthio ] -2 éthyl ] oxyde-1 thiadiazole [ 1, 2,5 ] diamine-3,4 ;

. N- [ [ (n. Butyl-5 dihydro-5,6-4H-thiéno [ 2,3-c ] pyrrolyl-2) méthylthio ] -2 éthyl ] oxyde-1 thiadiazole [ 1,2,5 ] diamine-3,4 ;

. N- [ [ (Isobutyl-5 dihydro-5,6-4H-thiéno [ 2,3-c ] pyrrolyl-2) méthylthio ] -2 éthyl ] oxyde-1 thiadiazole [ 1,2,5 ] diamine-3,4 ;

. N- [ [ (n.Hexyl-5 dihydro-5,6-4H-thiéno [ 2,3-c ] pyrrolyl-2) méthylthio ] -2 éthyl ] oxyde-1 thiadiazole [ 1,2,5 ] diamine-3,4 ;

. N- [ [ (Dihydro-5,6 n. pentyl-5-4H-thiéno [ 2,3-c ] pyrrolyl-2) méthylthio ] -2 éthyl ] oxyde-1 thiadiazole [ 1,2,5 ] diamine-3,4 ;

. N- [ [ [ Dihydro-5,6 (méthoxy-2 éthyl)-5-4H-thiéno [ 2,3-c ] pyrrolyl-2] méthylthio ] -2 éthyl ] oxyde-1 thiadiazole [ 1,2,5 ] diamine-3,4 ;

. Amino-3 [ [ (dihydro-5,6 n. propyl-5-4H-thiéno [ 2,3-c ] pyrrolyl-2) méthylthio ] -2 éthylamino ] -4 cyclobutène dione-1,2.

3. Sels d'addition des composés selon la revendication 1 ou 2, résultant de l'addition avec un acide minéral ou organique, tels les sels d'acides minéraux acceptables en thérapeutique, les chlorhydrates, bromhydrates, sulfates, phosphates, sulfonates, et les sels organiques particulièrement utilisés, les acétates, tartrates, citrates, camphosulfonates, maléates, fumarates, méthanesulfonates.

4. Procédé de préparation des composés selon la revendication 1, dans lesquels U est choisi parmi les groupements :

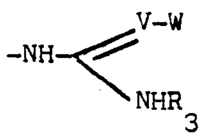

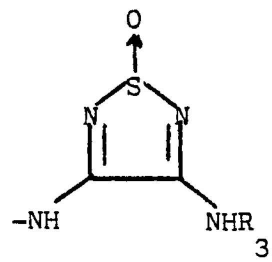

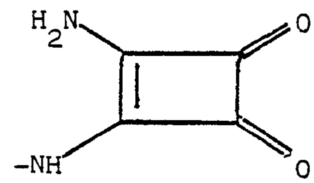

a la même signification que précedemment, caractérisé en ce qu'on fait réagir une amine de formule générale,

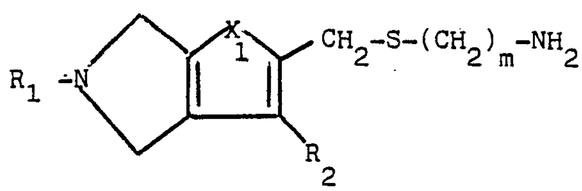

dans laquelle $R_1$, $R_2$, $X_1$ et m ont les mêmes significations que précédemment, respectivement avec un composé de formule :

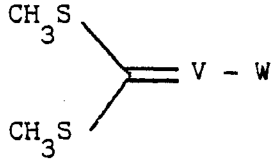

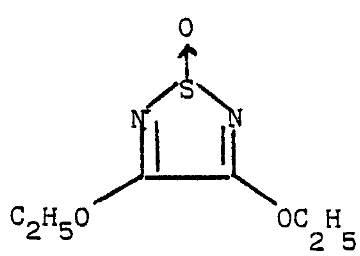

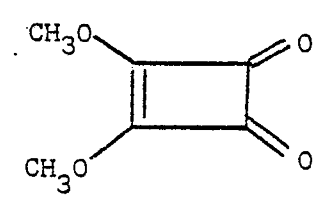

puis le composé ainsi obtenu avec une amine $R_3NH_2$, dans laquelle $R_3$ a la même signification que précédemment.

5 - Composés intermédiaires dans la préparation des composés de la revendication 1, de formule :

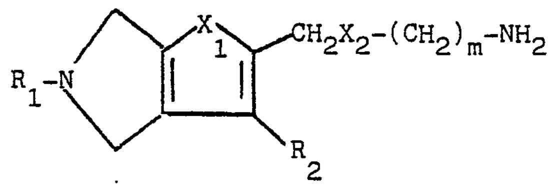

dans laquelle $R_1$, $R_2$, $X_1$, $X_2$ et m ont les significations précédemment définies avec la condition que lorsque $X_1$ représente un atome d'oxygène, $R_2$ représente un atome d'hydrogène, $X_2$ un atome de soufre et m égale 2.

6 - Procédé de préparation des composés selon la revendication 1, dans la formule desquels $X_1$ et $X_2$ désignent un atome de soufre, m = 2 et U un reste 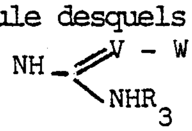

V,W ayant les mêmes significations que précédemment, caractérisé en ce qu'on fait réagir un dérivé halogéné de formule :

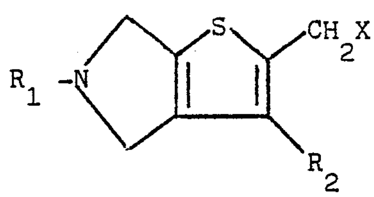

dans laquelle, X est un halogène, $R_1$, $R_2$ ont les mêmes significations que précédemment, ou l'un de ses sels avec un thiol de formule :

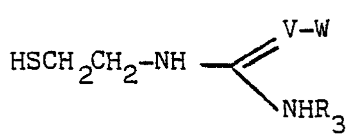

dans laquelle V, W et $R_3$ ont les mêmes significations que précédemment.

7 - Procédé de préparation des composés selon la revendication 1, dans la formule desquels $X_1$ et $X_2$ désignent un atome de soufre, m = 2 et U représente un reste aminosulfonylamidine, caractérisé en ce qu'on

fait réagir un iminoéther de formule :

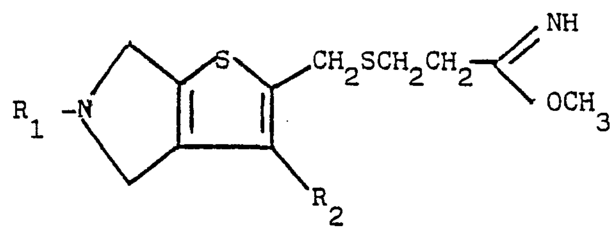

dans laquelle $R_1$ et $R_2$ ont les mêmes significations que précédemment avec le sulfamide.

8. Procédé de préparation des composés selon la revendication 1 dans la formule desquels $R_2$ désigne l'hydrogène et U désigne un reste méthyl-1-1H-diamino-3,5 triazole-1,2,4, caractérisé en ce qu'on fait réagir un composé de formule :

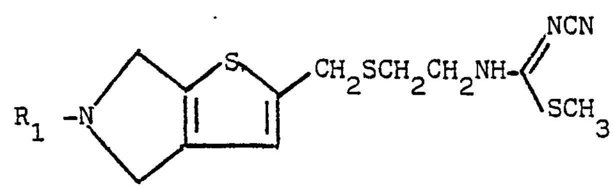

avec la méthylhydrazine.

9. Composés intermédiaires dans la préparation des composés selon la revendication 1, quand $X_1$ est un atome de soufre, représentés par la formule générale

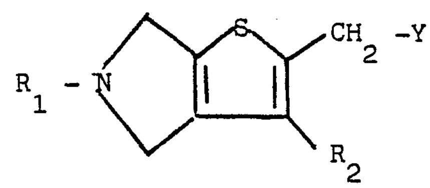

dans laquelle $R_1$ et $R_2$ ont les significations précédemment définies et Y désigne les groupements OH, halogène, SH, $-S\ (CH_2)_2CN$, et

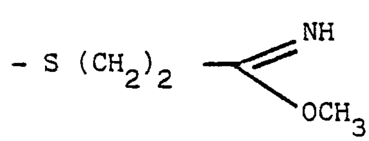

10. Composés intermédiaires dans la préparation des composés selon la revendication 1, quand $X_1$ est un atome d'oxygène, représentés par la formule :

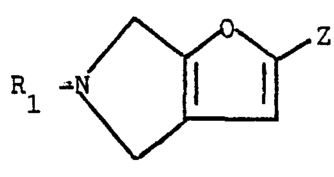

dans laquelle $R_1$ a les significations énoncées précédemment et Z désigne un groupement : $-COOCH_3$ ou $-CH_2OH$.

11. Médicament contenant comme principe actif un composé selon une quelconque des revendications 1 à 3, en mélange avec un excipient pharmaceutique.

12. Médicament selon la revendication 11, sous forme d'unité de dosage dans laquelle chaque unité de dosage contient de 5 à 500 mg de principe actif en mélange avec un excipient pharmaceutique.

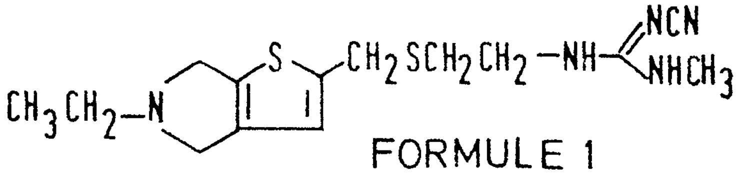

FORMULE 1

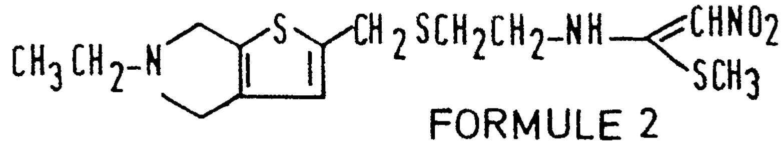

FORMULE 2

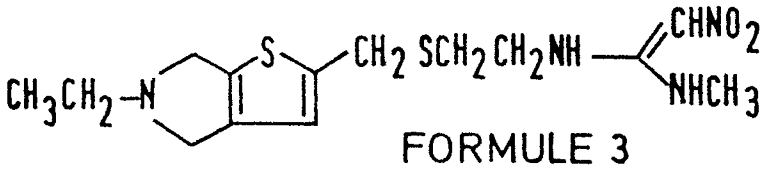

FORMULE 3

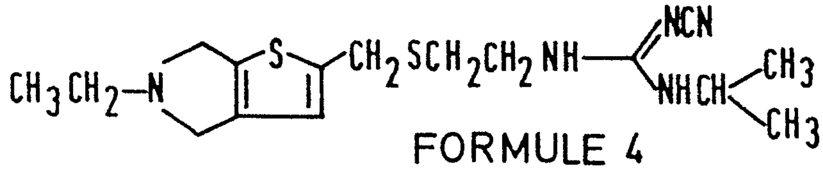

FORMULE 4

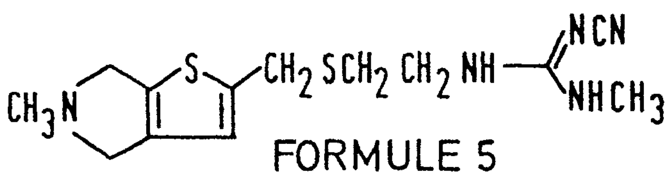

FORMULE 5

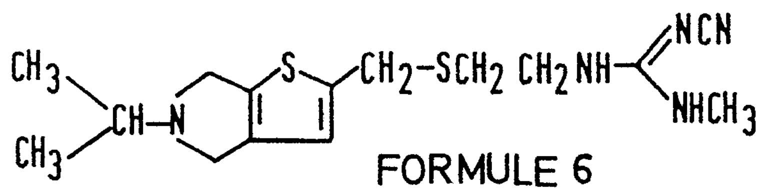

FORMULE 6

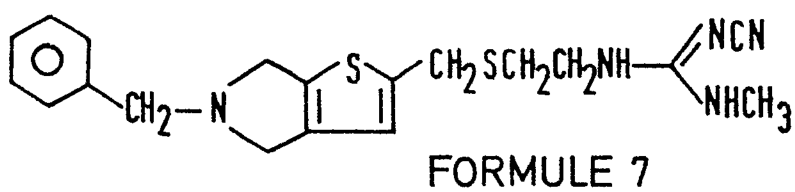

FORMULE 7

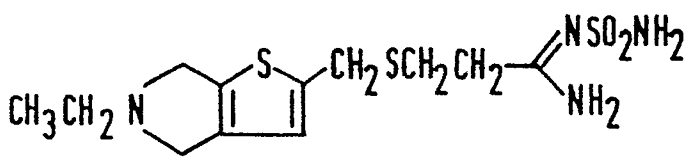

FORMULE 8

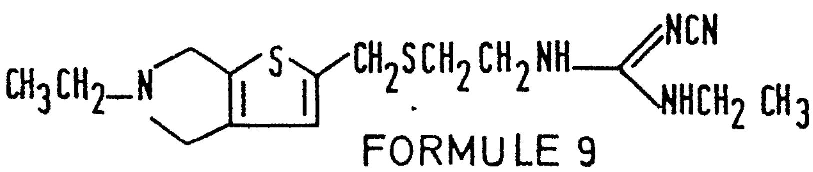

FORMULE 9

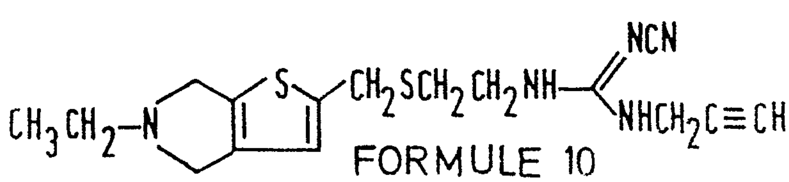

FORMULE 10

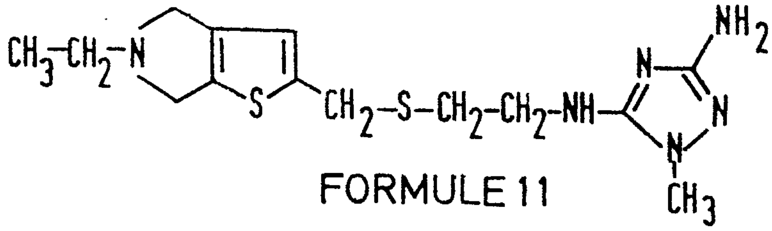

FORMULE 11

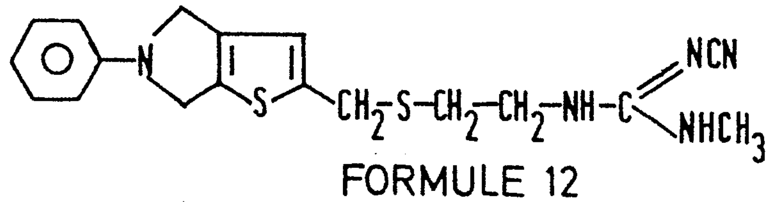

FORMULE 12

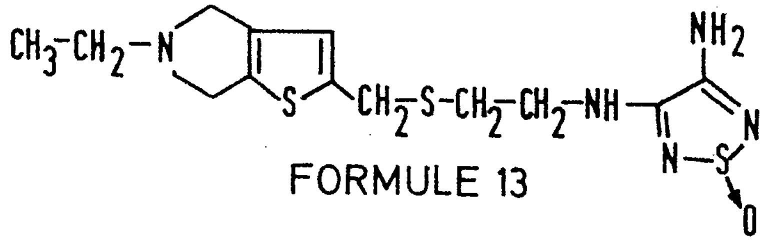

FORMULE 13

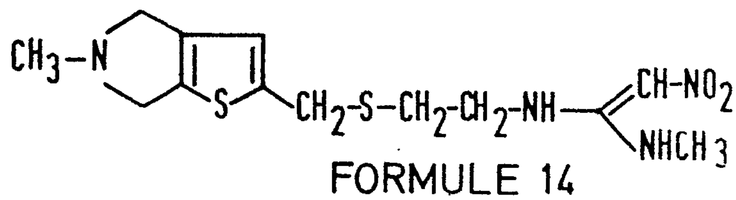

FORMULE 14

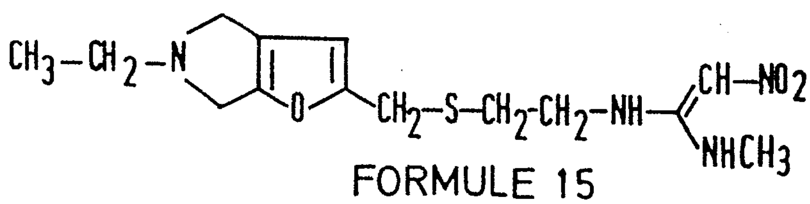

FORMULE 15

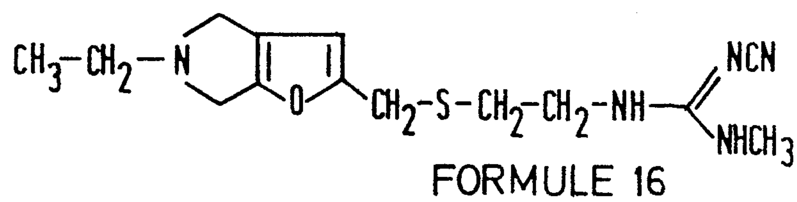

FORMULE 16

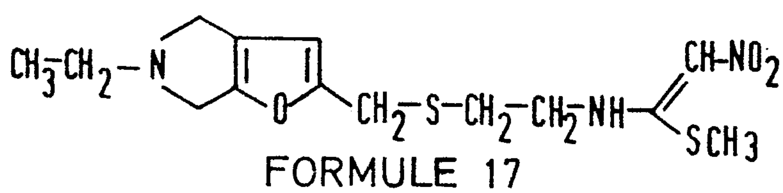

FORMULE 17

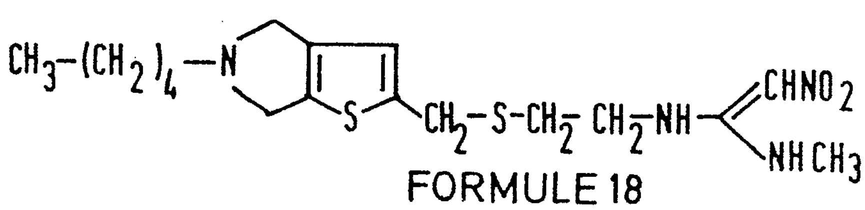

FORMULE 18

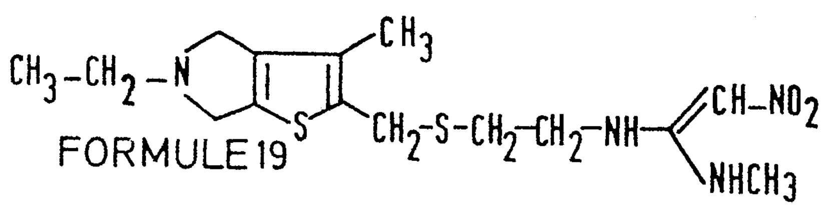

FORMULE 19

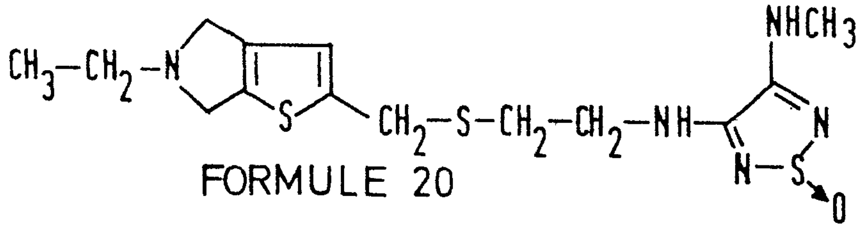

FORMULE 20

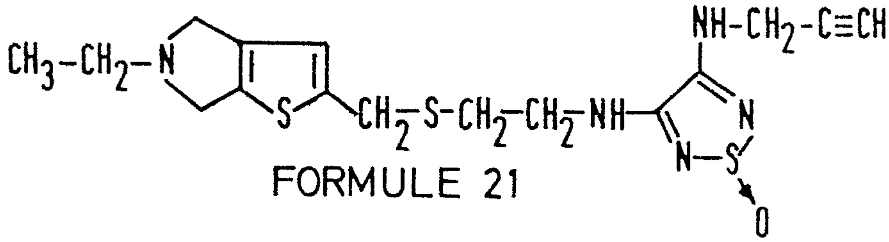

FORMULE 21

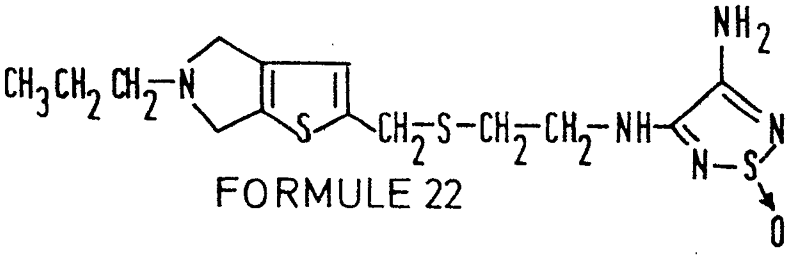

FORMULE 22

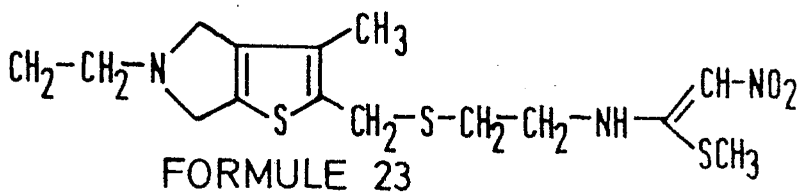

FORMULE 23

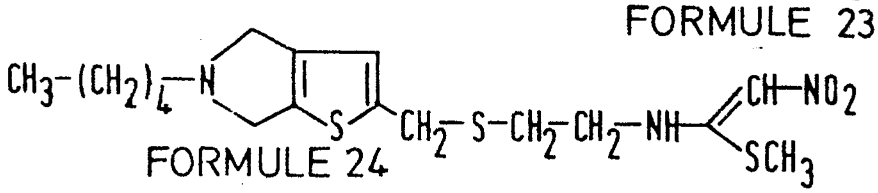

FORMULE 24

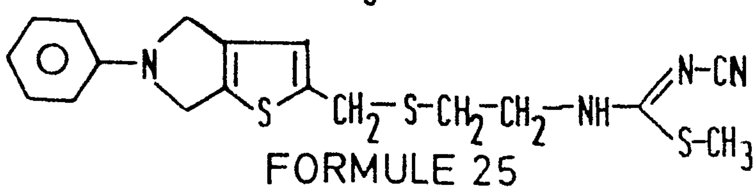

FORMULE 25

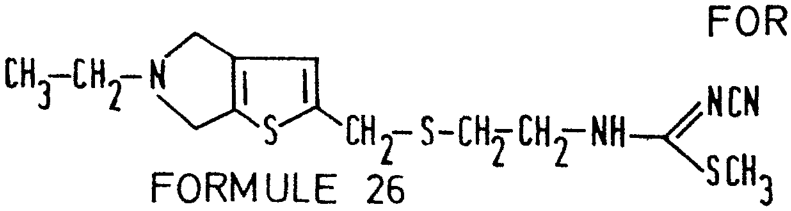

FORMULE 26

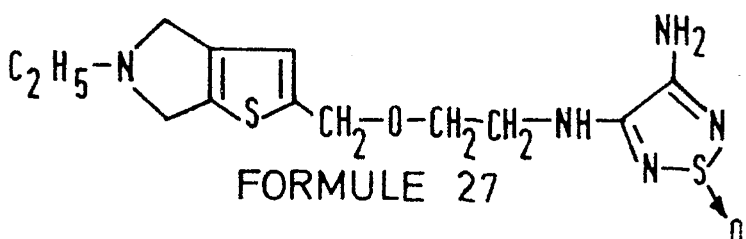

FORMULE 27

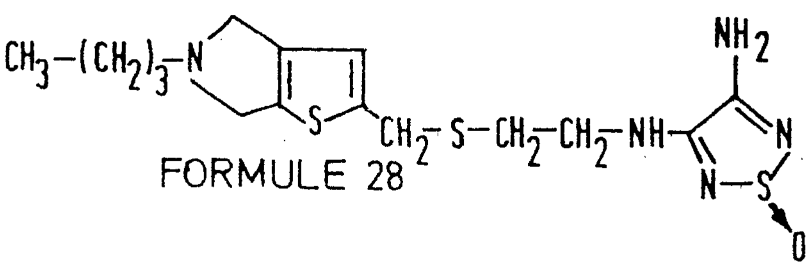

FORMULE 28

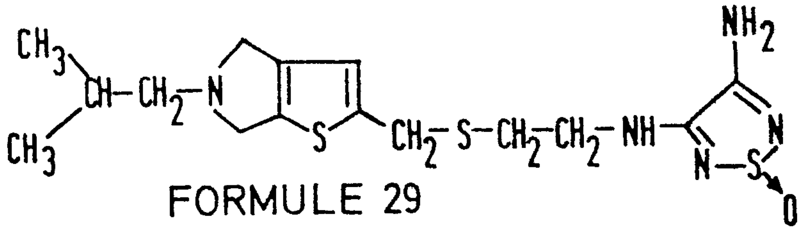
FORMULE 29

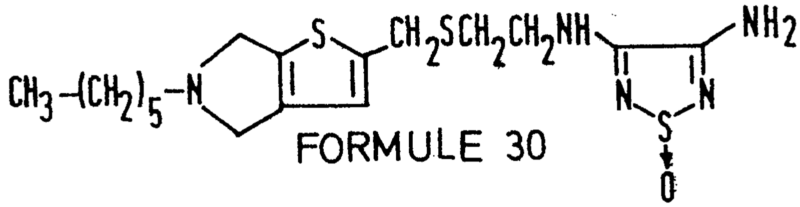
FORMULE 30

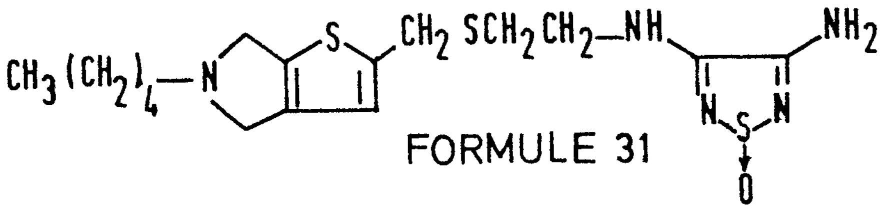
FORMULE 31

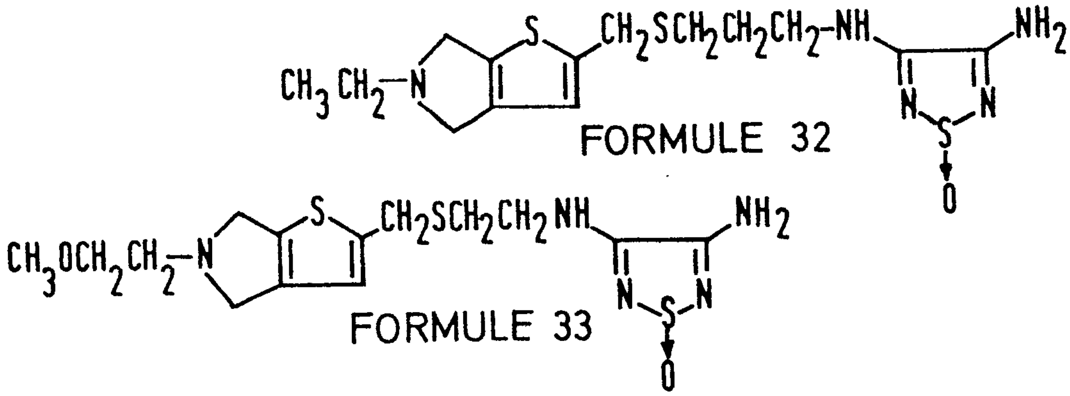
FORMULE 32

FORMULE 33

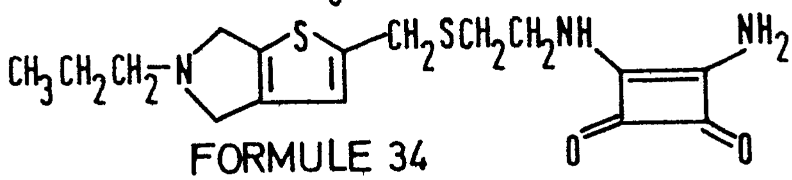
FORMULE 34

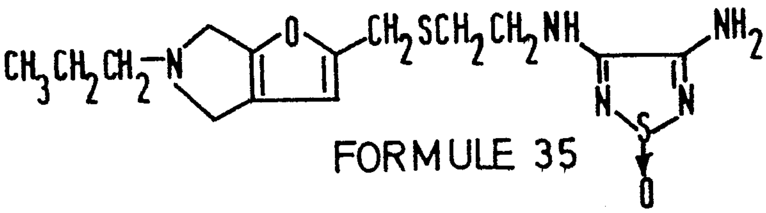
FORMULE 35

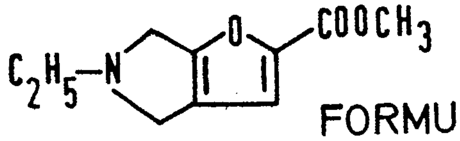
FORMULE 36

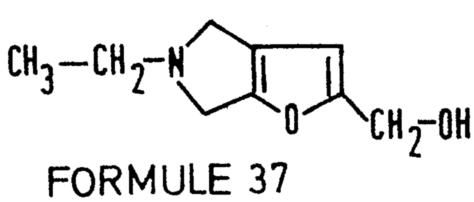
FORMULE 37

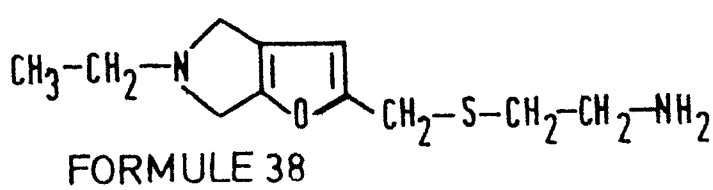
FORMULE 38

0180500

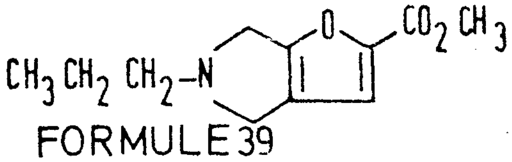
FORMULE 39

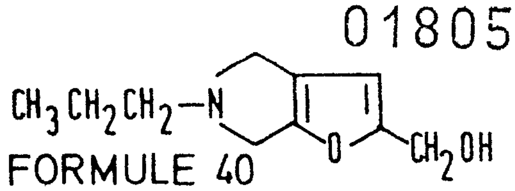
FORMULE 40

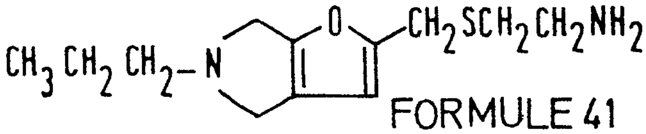
FORMULE 41

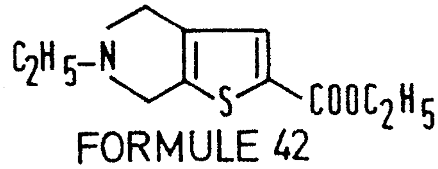
FORMULE 42

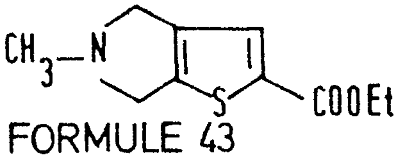
FORMULE 43

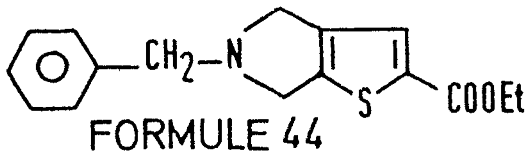
FORMULE 44

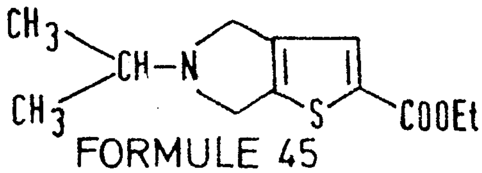
FORMULE 45

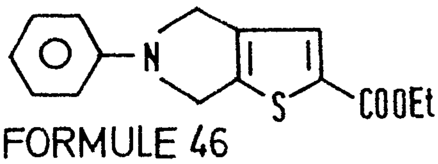
FORMULE 46

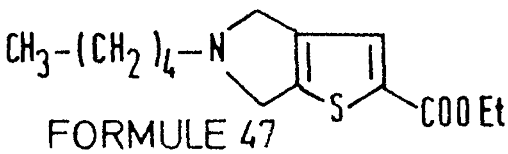
FORMULE 47

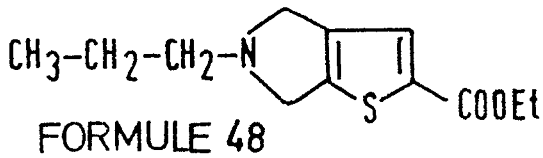
FORMULE 48

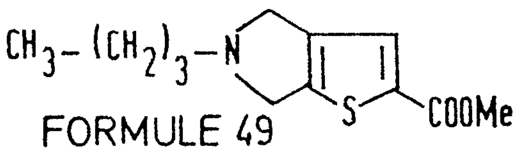
FORMULE 49

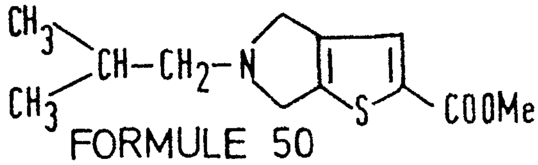
FORMULE 50

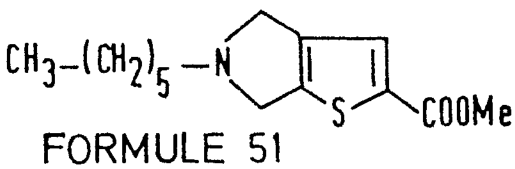
FORMULE 51

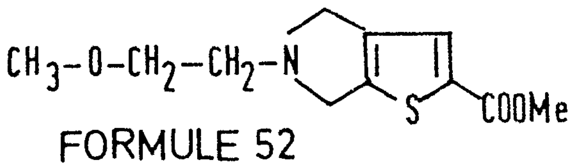
FORMULE 52

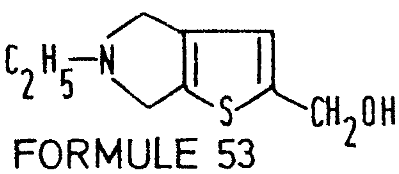
FORMULE 53

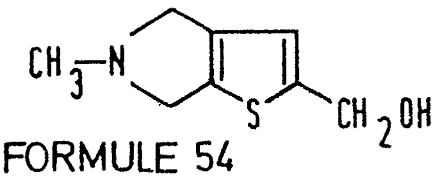
FORMULE 54

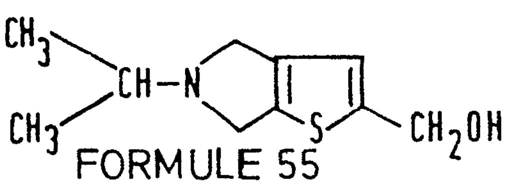
FORMULE 55

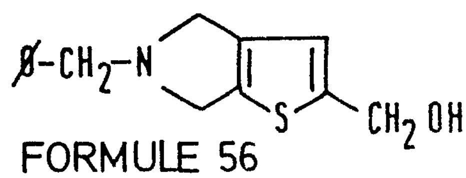
FORMULE 56

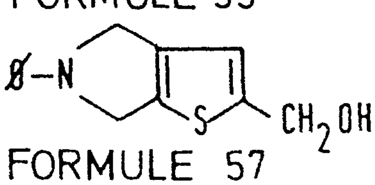
FORMULE 57

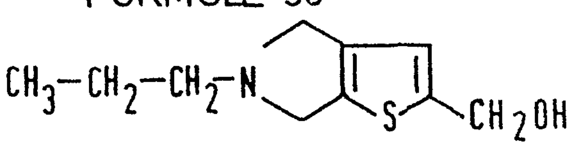
FORMULE 58

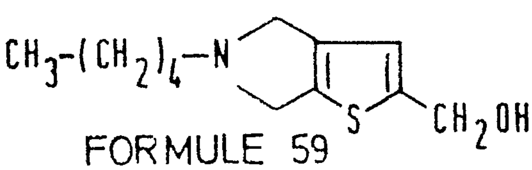
FORMULE 59

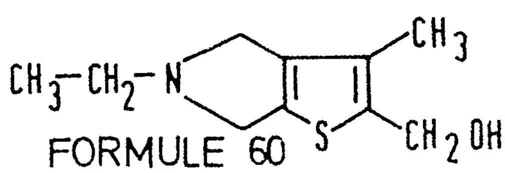
FORMULE 60

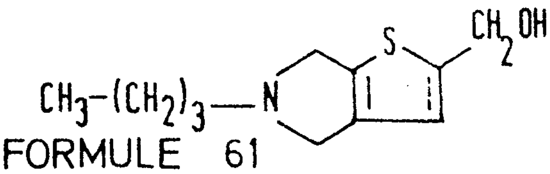

FORMULE 61

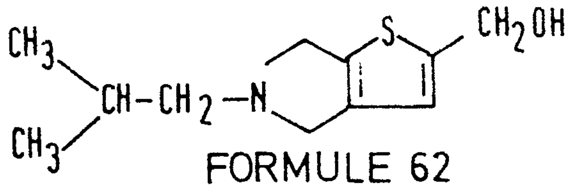

FORMULE 62

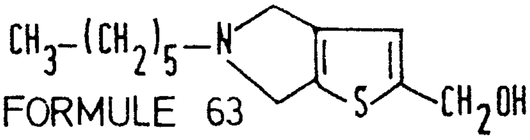

FORMULE 63

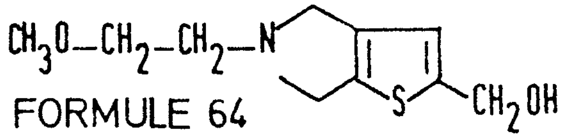

FORMULE 64

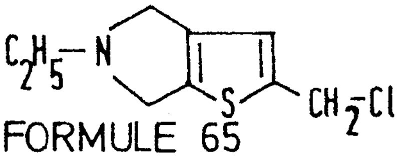

,HCl

FORMULE 65

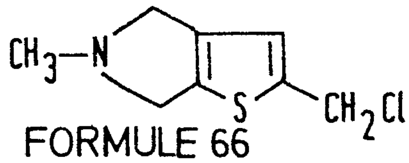

,HCl

FORMULE 66

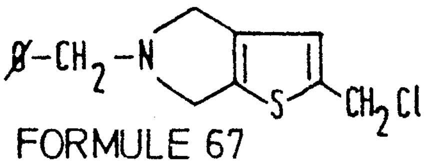

,HCl

FORMULE 67

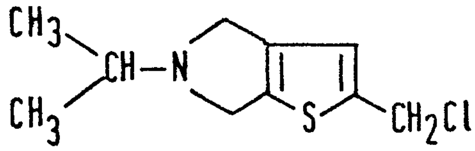

,HCl

FORMULE 68

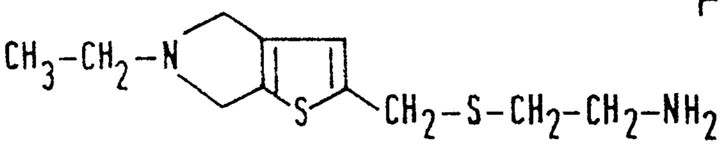

FORMULE 69

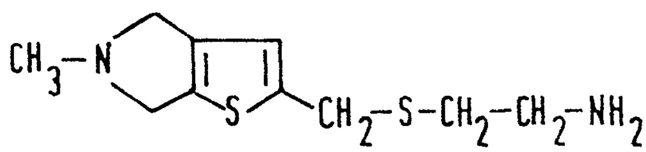

FORMULE 70

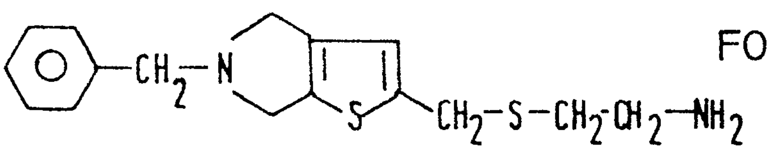

FORMULE 71

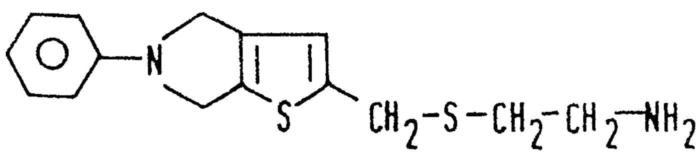

FORMULE 72

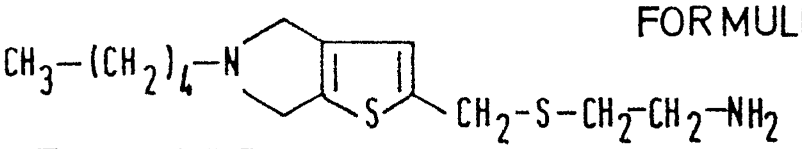

FORMULE 73

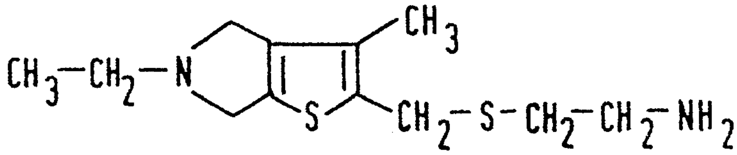

FORMULE 74

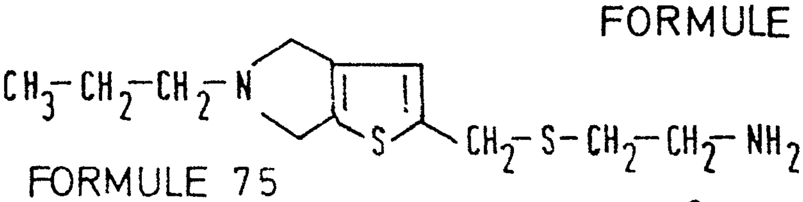

FORMULE 75

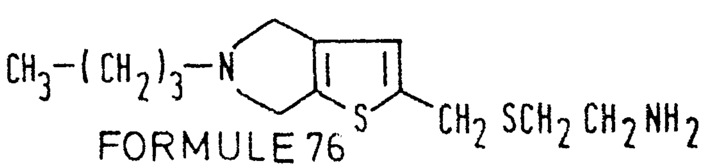

FORMULE 76

0180500

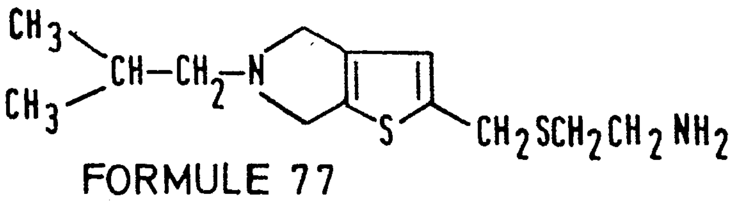

FORMULE 77

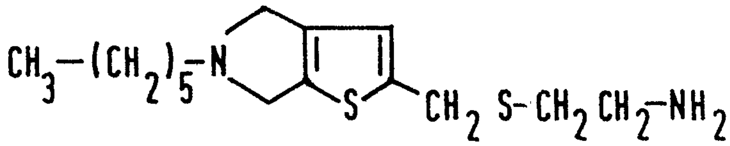

FORMULE 78

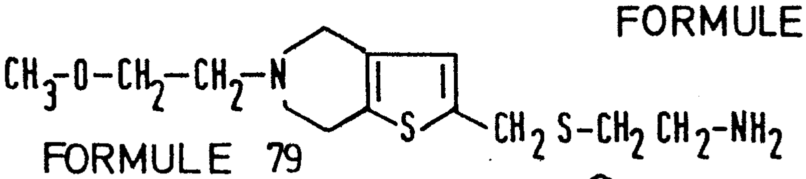

FORMULE 79

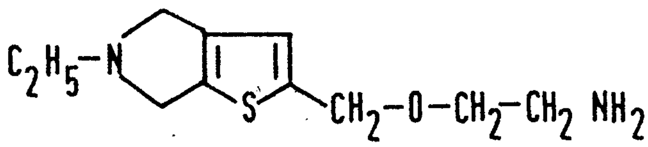

FORMULE 80

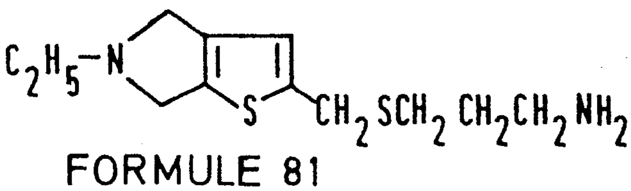

FORMULE 81

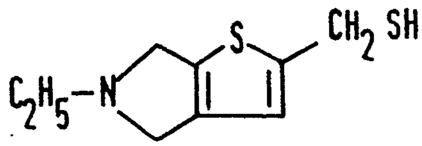

FORMULE 82

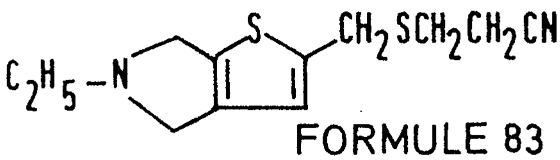

FORMULE 83

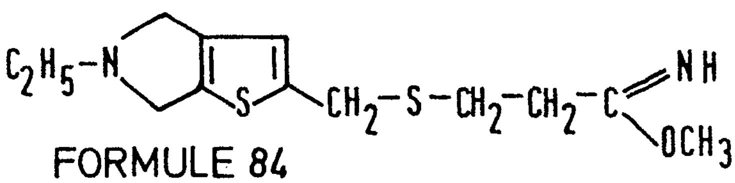

FORMULE 84

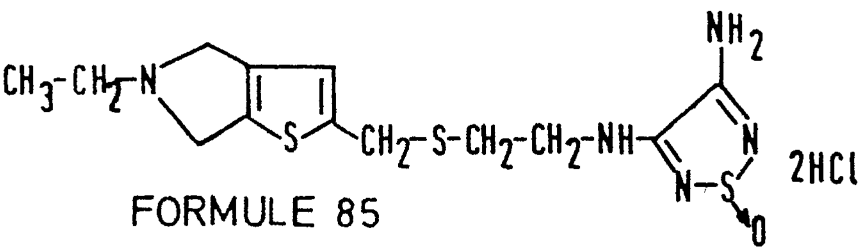

FORMULE 85

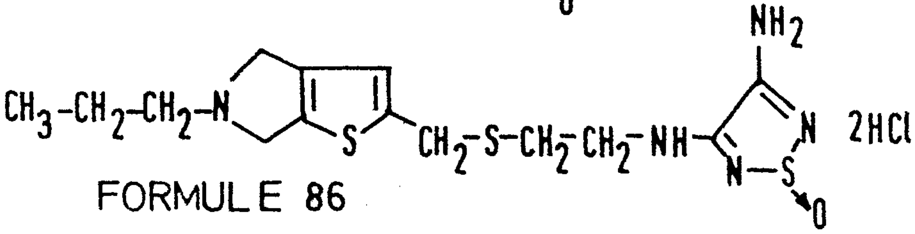

FORMULE 86

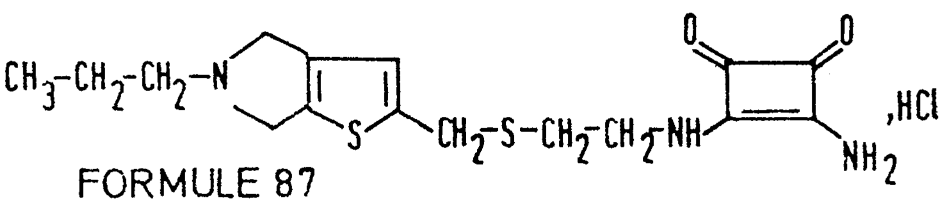

FORMULE 87

0180500

Office européen des brevets

# RAPPORT DE RECHERCHE EUROPEENNE

Numero de la demande

EP 85 40 1941

## DOCUMENTS CONSIDERES COMME PERTINENTS

| Catégorie | Citation du document avec indication, en cas de besoin, des parties pertinentes | Revendication concernee | CLASSEMENT DE LA DEMANDE (Int. Cl.4) |
|---|---|---|---|
| D,Y | WO-A-8 400 544 (RORER)<br>* Revendications 1,29 * | 1,11 | C 07 D 491/04<br>C 07 D 495/04<br>A 61 K 31/40<br>A 61 K 31/41 //<br>(C 07 D 491/04<br>C 07 D 307:00<br>C 07 D 209:00 )<br>(C 07 D 495/04<br>C 07 D 333:00<br>C 07 D 209:00 ) |
| D,Y | FR-A-2 384 765 (ALLEN & HANBURYS)<br>* Revendication 1; page 1, lignes 20-31 * | 1,11 | |
| D,Y | FR-A-2 391 209 (ALLEN & HANBURYS)<br>* Revendication 1; page 1,, lignes 20-36 * | 1,11 | |
| D,Y | GB-A-2 098 988 (BRISTOL-MYERS)<br>* Revendication 1; page 8, lignes 54-64; page 9 * | 1,11 | DOMAINES TECHNIQUES RECHERCHES (Int. Cl.4)<br>C 07 D 491/00<br>C 07 D 495/00<br>A 61 K 31/00 |

Le présent rapport de recherche a été établi pour toutes les revendications

| Lieu de la recherche | Date d'achèvement de la recherche | Examinateur |
|---|---|---|
| LA HAYE | 13-01-1986 | ALFARO I. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire
T : théorie ou principe à la base de l'invention
E : document de brevet antérieur, mais publié à la date de dépôt ou après cette date
D : cité dans la demande
L : cité pour d'autres raisons
& : membre de la même famille, document correspondant

OEB Form 1503 03 82